(19) Europäisches Patentamt
European Patent Office
Office européen des brevets

(11) Publication number: 0 009 362
B1

(12) EUROPEAN PATENT SPECIFICATION

(45) Date of publication of patent specification: 27.06.84

(21) Application number: 79301860.7

(22) Date of filing: 11.09.79

(51) Int. Cl.[3]: C 07 D 233/48, C 07 D 233/88, C 07 D 213/75, C 07 D 215/46, C 07 D 263/28, C 07 D 265/08, C 07 D 277/48, C 07 D 279/06, C 07 D 405/12, C 07 D 407/12 //C07D413/12, C07D417/12, A61K31/415, A61K31/42, A61K31/425, A61K31/445, A61K31/47, (C07D213/75, 213/64), (C07D215/46, 215/22)

# (54) Heterocyclic derivatives of guanidine, their preparation and pharmaceutical formulations comprising them.

(30) Priority: 18.09.78 US 943097
18.09.78 US 943098
18.09.78 US 943099

(43) Date of publication of application:
02.04.80 Bulletin 80/7

(45) Publication of the grant of the patent:
27.06.84 Bulletin 84/26

(84) Designated Contracting States:
AT BE CH DE FR GB IT NL SE

(56) References cited:
BE - A - 852 565
FR - A - 2 181 714
FR - A - 2 285 879
FR - M - 8 382
US - A - 3 352 878

The file contains technical information submitted after the application was filed and not included in this specification

(73) Proprietor: McNeilab, Inc.
Camp Hill Road
Fort Washington Pennsylvania 19034 (US)

(72) Inventor: Rasmussen, Chris Royce
1540 Butler Pike
Ambler Pennsylvania (US)

(74) Representative: Jones, Alan John et al,
CARPMAELS & RANSFORD 43 Bloomsbury Square
London, WC1A 2RA (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Background of the invention

In British Patent No. 1,409,768 there are described several heterocyclic derivatives of guanidine in which the heterocyclic moiety is a 5- or 6-membered saturated 1,3-diazocarbocyclic-2-ylidene. These derivatives are unsubstituted on the imino nitrogen of the guanidine moiety. In contrast, the compounds of the present invention differ by being a heterocyclic derivative of guanidine which carries a bulky substituent on the imino nitrogen of the guanidine moiety. In Belgian Patent No. 852,565 there are described derivatives of guanidine which carry a bulky substituent on the imino nitrogen of the guanidine moiety, which derivatives, in contrast to the compounds of the present invention, are saturated mono-aza heterocyclic derivatives. Additional prior art, but further related, may be represented by French Pat. No. 8382M, German Offen. Nos. 2,321,330 and 2,502,397; U.S. Patent Nos. 3,914,306, 3,933,836 and 4,073,636; and British Patent No. 1,341,245.

Description of the preferred embodiments:

This invention relates to new heterocyclic derivatives of guanidine having interesting pharmacological properties and, more particularly, to such derivatives having the formula

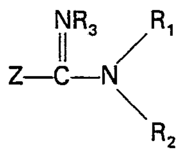

(I)

wherein

Z is a member selected from:

(a) 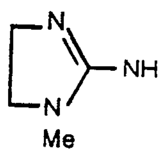

[Tautomeric form: 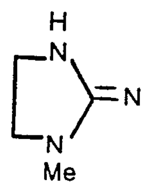 ],

(b) 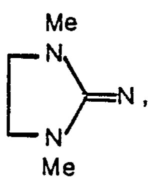, (c) 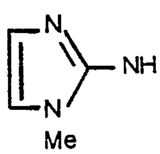 (d) 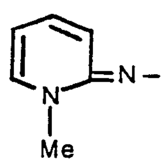

(e) 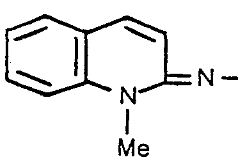 and (f) 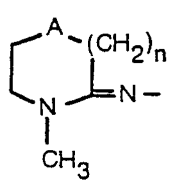

wherein:

A is O or S; n is zero or 1;

$R_1$ is methyl or ethyl;

$R_2$ is $C_1$—$C_4$ alkyl (preferably methyl and ethyl), cycloalkyl having from 3 to 6 carbons (preferably cyclopentyl and cyclohexyl) or;

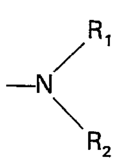

taken together represents

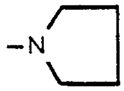 , 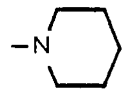 , or 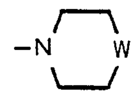

wherein

W is O, W, N-$C_1$—$C_4$ alkyl (preferably N-methyl) or N-phenyl; and

$R_3$ is alklyl having from 4 to 10 carbons (preferably branched), such as, for example, *tert.*-butyl, neopentyl or 1,1,3,3-tetramethylbutyl (*tert.*-octyl); phenyl; methylenedioxyphenyl; phenyl substituted with from 1 to 3 substituents each selected from halo, $C_1$—$C_4$ alkyl and $C_1$—$C_4$ alkoxy; and phenyl substituted with hydroxy, benzyloxy, nitro; trifluoromethyl or methylthio; naphthyl; cycloalkyl having from 5 to 8 carbons (preferably cyclopentyl and cyclohexyl); bicycloalkyl having from 7 to 10 carbons, such as for example, *exo*- and *endo*-2-norbornyl, 2-bicyclo[2.2.2.]-octyl or *endo*-2-bicyclo[3.2.1.]octyl; tricycloalkyl having 9 or 10 carbons, such as, for example, *nor*-adamantyl, 1- and 2-adamantyl or 1-and 2-(2,3,3a,4,5,6,7,7a-octahydro-4,7-methanoindenyl); arylalkyl in which the aryl function is phenyl or naphthyl and the alkyl function has from 1 to 4 carbons, such as, for example, benzyl, *dl*-, *d*- or *l*-$\alpha$-phenethyl, *dl, d*- or *l*-$\alpha$-methylbenzyl, $\alpha,\alpha$-dimethylbenzyl, $\alpha,\alpha$-dimethyl-$\beta$-phenethyl, or *dl, d*- or *l*-($\alpha$-naphthyl)ethyl; or diphenylalkyl in which the alkyl function has 1 or 2 carbons, such as, for example, diphenylmethyl, or 1,2- and 2,2-diphenylethyl.

As used herein, the term "halo" represents halogens of atomic weight less than 127, i.e., chloro, bromo, fluoro, and iodo.

Due to the presence of the amine-like nitrogen atoms in the compounds of formula (I), acid addition salts thereof are readily obtained and such pharmaceutically acceptable salts are included within the scope of this invention. The subject compounds (I) may be converted to their therapeutically active nontoxic acid addition salt form by treatment with an appropriate acid, such as, for example, an inorganic acid, such as hydrohalic acid, e.g., hydrochloric or hydrobromic and sulfuric acid, nitric acid or phosphoric acid or an organic acid, such as, for example, acetic, propionic, glycolic, pamoic, pyruvic, malonic, succinic, maleic, fumaric, malic, tartaric, citric, benzoic, cinnamic, mandelic, methanesulfonic, ethanesulfonic, benzenesulfonic, *p*-toluenesulfonic, cyclohexanesulfamic, salicyclic or *p*-amino-salicyclic. Conversely, the salt form can be converted by treatment with alkali into the free base form.

Preferred compounds in accordance with the present invention include:

(a) N-(1-methyl-2-imidazolidinylidene)-N'-phenyl-1-pyrrolidinecarboximidamide;
(b) N-(1,3-dimethyl-2-imidazolidinylidene)-N'-phenyl-1-pyrrolidinecarboximidamide;
(c) N-(1-methylimidazol-2-yl)-N'-phenyl-4-morpholine-carboximidamide;
(d) N-(1-adamantyl)-N'-(1-methylimidazol-2-yl)-1-pyrrolidinecarboximidamide;
(e) N-(1,2-dihydro-1-methyl-2(1*H*)pyridinylidene)-N'-phenyl-1-pyrrolidinecarboximidamide;
(f) N-(1,2-dihydro-1-methyl-2(1*H*)quinolinylidene)-N'-phenyl-1-pyrrolidinecarboximidamide;
(g) N-(3-methyl-2-thiazolidinylidene)-N'-phenyl-1-pyrrolidinecarboximidamide;
(h) N-(3-methyl-2-oxazolidinylidene)-N'-phenyl-1-pyrrolidinecarboximidamide;
(i) N-(2,3,5,6-tetrahydro-4-methyl-1,4-oxazine-3-ylidene)-N'-phenyl-1-pyrrolidinecarboximidamide;
(j) N-[(2,3,5,6-tetrahydro-4-methyl)-1,4-thiazin-3-ylidene]-N'-(4-methoxyphenyl)-1-piperidine-carboximidamide;

and the pharmaceutically acceptable acid addition salts thereof.

The subject compounds of formula (I) may be prepared from the starting materials of formula (II), wherein $Z_1$ is the same as Z, but other than (d) or (e), and in the case of (f) A is sulfur and n is zero. Said starting materials ($Z_1H$) may be reacted with an isothiocyanate of formula (III), wherein $R_3$ is as previously described other than hydroxyphenyl, and in the instance wherein $Z_1$ is (f), $R_3$ is as previously described, but other than hydroxyphenyl and $C_1$—$C_4$ alkanoyloxyphenyl, in a reaction-inert organic solvent, e.g., benzene, $CH_2Cl_2$ or chloroform at temperatures ranging from about ambient to reflux temperatures, for about 2 to 24 hours, in approximately equimolar amounts. The thio function (=S) in the thus-obtained thioureas (IV), is then transformed into an alkylthio function (—SR') by reacting (IV) with an alkylating agent of the formula R'X, wherein R' is ethyl or, preferably, methyl, and X is halide, preferably iodide, tosylate, methosulfate, mesylate or fluorosulfonate. Typical solvents for such alkylations include ethers, preferably diethyl ether, tetrahydrofuran, or dioxane, lower ketones, e.g., acetone or 2-butanone; halohydrocarbons and $C_1$—$C_4$ alkanols, preferably methylene dichloride and methanol, respectively. Methyl iodide as the alkylating agent in methanol is particularly suitable. Generally, equimolar to a large stoichiometric excess of the alkylating agent is used, the amount depending on the reactivity of the thiourea (IV) or its solubility in the solvent employed. The alkylation reaction may be carried out at temperatures ranging from ambient to reflux or in appropriate sealed vessels at higher temperatures. The alkylthio compounds of formula (V) in acid addition (HX) salt form are then reacted with an appropriate amine of the formula $HNR_1R_2$, wherein $R_1$, $R_2$ and $NR_1R_2$ are as previously described, preferably in a $C_1$—$C_4$ alkanol solvent such as isopropanol and *tert.*-butanol and generally at reflux temperatures of about 40—100°C., to yield the guanidine derivatives of formula (I),

in similar acid addition form, which are readily obtained as the corresponding base form by conventional treatment with suitable alkali. The foregoing reactions may be illustrated as follows:

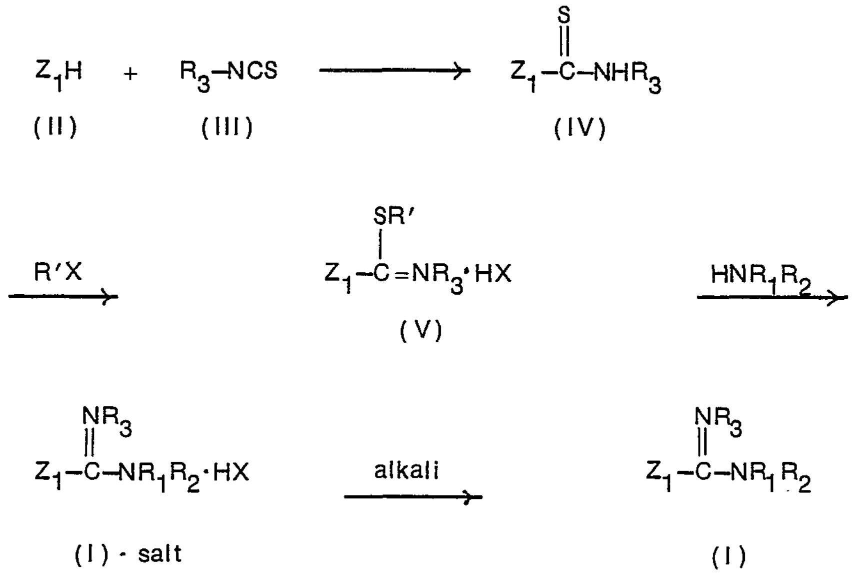


(II) (III) (IV) (V) (I) - salt (I)

The isothiocyantes of formula (III), many of which are known, may be prepared according to the extensive processes reported in the literature for making isothiocyantes. For example, they may be obtained from the methodologies reported by M. Bögemann et al. in *Methoden der Organische Chemie Houben-Weyl,* Eugen Müller (Ed.), Georg Thieme Verlag (Publ.) Stuttgart, Germany, Vol. 9, page 867—884 (1955); *Preparation des Isothiocyanates Aromatiques* by A. Rasschaert et al., Ind. Chim., Belge, *32*, 106 (1967); German Patent No. 1,300,559; J. Org. Chem., *36*, 1549 (1971); U.S. Pat. Nos. 2,395,455 and 3,304,167; French Pat. No. 1,528,249; "A New Synthesis of Aliphatic Isothiocyantes" Angew. Chem. Internat. Ed., *6*, 174 (1967); Bull. Chem. Soc. Japan, *48*, 2981 (1975); Tetrahedron, *29*, 691 (1973); Chem. Ber., *101*, 1746 (1968); and J. Indian Chem. Soc., *52*, 148 (1975).

In the foregoing reaction of (V) with the amine, $HNR_1R_2$, it is preferred to use a stoichiometric excess of the latter, for example, in 1:1.05 to 1:2.0 molar ratios. If only a slight excess of the $HNR_1R_2$ amine is used, it may be advantageous to add a stoichiometric equivalent of a tertiary alkyl amine, e.g., $Et_3N$, in order to enhance the rate of reaction. Any by-products which may be formed during the course of the reaction can be separated from the desired formula (I) product by standard techniques known in the art, such as, for example, by fractional solubilization.

In each of the foregoing synthetic procedures for preparing formula (I) compounds wherein $Z_1$ is (f), hydroxyphenyl and $C_1$—$C_4$ alkanoyloxyphenyl were excluded from the original definition of $R_3$. The formula (I) compounds wherein $Z_1$ is (f) and wherein $R_3$ is hydroxyphenyl may be prepared by hydrolysis of the corresponding $R_3$=methoxyphenyl derivatives by conventional procedures, e.g., by treatment with HBr or HI and acetic acid. Acylation of the resultant $R_3$=hydroxyphenyl derivatives by glacial acetic acid in the presence of excess dicyclohexylcarbodiimide affords the corresponding $R_3$=$C_1$—$C_4$ alkanoyloxy derivatives of formula (I) [and in which $Z_1$ is (f)].

The compounds of formula (I), wherein $R_1$, $R_2$, $NR_1R_2$ and $R_3$ (other than hydroxyphenyl) are as previously defined and Z is equal to the functions represented by letters (b), (d), (e), and (f), are alternatively prepared by reacting a pseudouronium salt of formula (VI), or a lactam salt of formula (XV) or (IXX) wherein X is either methoxy or ethoxy and $Y^{\theta}$ is either $BF_4^{\theta}$ or $OSO_2F^{\theta}$, with a guanidine derivative for formula (VII), with stoichiometric quantities of reactants being preferably employed.

It is often advantageous to add four to eight molar equivalents of potassium carbonate to the reaction mixture following addition of the guanidine (VII) in order to cause the reaction to proceed toward completion. Suitable anhydrous organic solvents for conducting the reaction include $C_1$—$C_4$ aliphatic alcohols, such as, for example, methanol, ethanol, 2-propanol or *tert.*-butanol; ethers, such as, for example, diethylether, tetrahydrofuran or dioxane; and lower halogenated hydrocarbons, such as, for example, chloroform, methylene chloride or 1,2-dichloroethane. Generally, methylene chloride is preferred for compound (VI) and *tert.*-butanol for compounds (XV) or (IXX). Ambient to 0°C. temperatures may generally be employed in the case of compounds (VI), and ambient to reflux temperatures (about 80°C.) are employed in the case of compounds (XV) or (IXX). The products (VIII), (XVI) or (XX), in the form of the corresponding HY salt, is converted to the corresponding base form (I) by conventional means, for example, by treatment with a suitable alkali such as alkali metal or alkaline earth metal hydroxides; or carbonates. The reactions may be illustrated as follows:

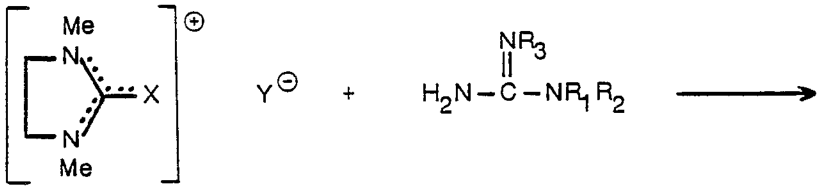

(VI) (VII)

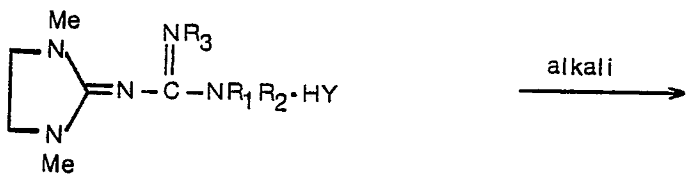


(I)

(VIII)

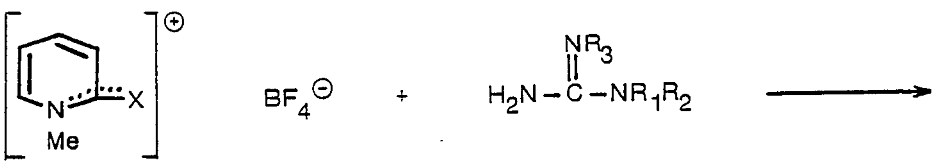

(XV) (VII)

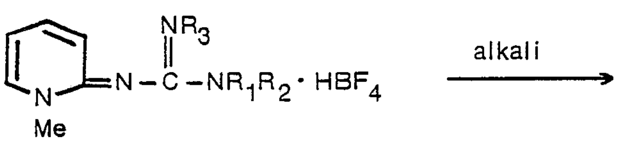


(I)

(XVI)

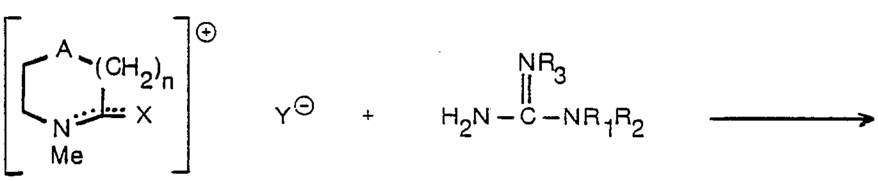

(IXX) (VII)

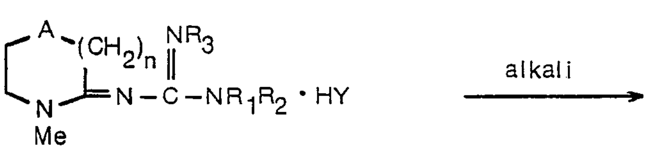


(I)

(XX)

The pseudouronium fluoborates of formula (VI), wherein $Y^{\theta}$ is $BF_4^{\theta}$, may be obtained according to procedures described in the literature, e.g., see Canadian Patent Nos. 850,116 and 950,464; U.S. Pat. No. 3,876,658; Ber. *89*, 2063 (1956); and Org. Synth. *46*, 113, 120 (1966). The fluorosulfonates of formula (VI-b), or (XXII-b) wherein $Y^{\theta}$ is $OSO_2F^{\theta}$, are similarly prepared. In general, a cyclic urea of formula (IX) or a lactam of formula (XVII-a) or (XVII-b) or an oxo compound of formula (XXI) is reacted with an appropriate trialkyl oxonium fluoroborate (X) or said compounds of formulae (IX) or (XXI) is reacted with methyl fluorosulfonate (XI) to give the corresponding salt (VI) or (XXII). The reaction is preferably carried out from 0°C. to ambient temperature under an inert dry atmosphere (e.g., nitrogen, argon) in an inert anhydrous lower halohydrocarbon solvent such as, for example, chloroform, 1,2-dichloroethane and methylene dichloride (most preferred). Other inert anhydrous organic solvents that may be employed include ethers such as, for example, diethyl ether, dioxane, tetrahydrofuran (THF) and 1,2-dimethoxyethane. The foregoing reactions may be illustrated as follows:

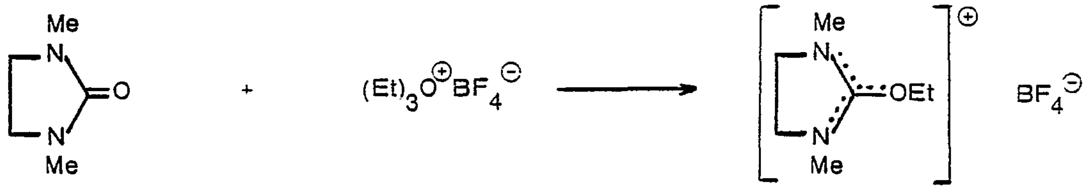

(IX) (X) (VI–a)

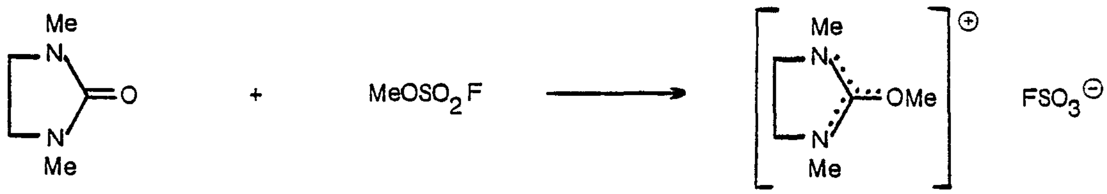

(IX) (XI) (VI–b)

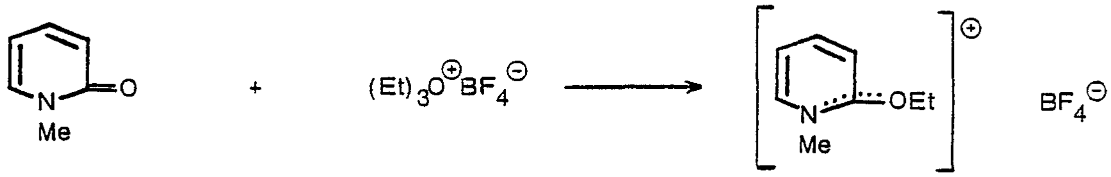

(XVII–a) (X) (XVII–a)

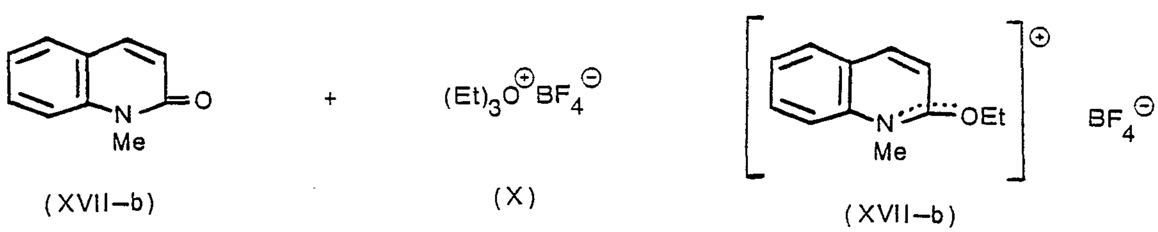

(XVII–b) (X) (XVII–b)

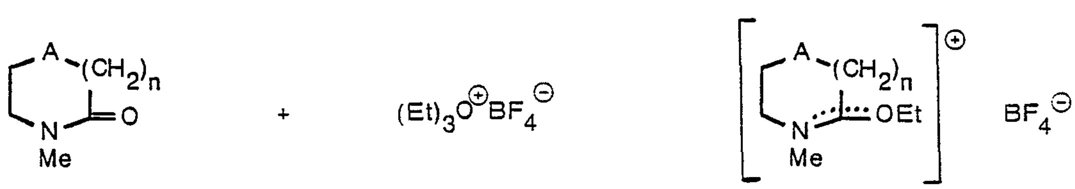

(XXI) (X) (XXII–a)

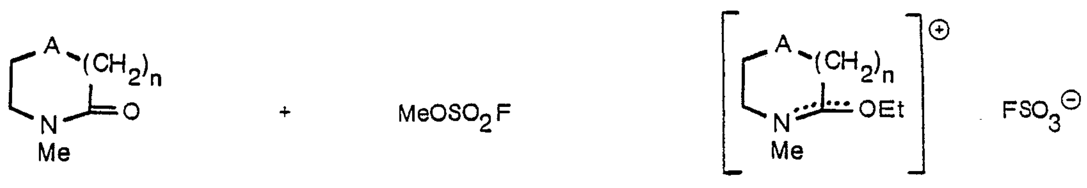

(XXI) (XI) (XXII-b)

In the foregoing synthetic procedures for preparing formula (I) compounds wherein Z is (d) or (e), hydroxyphenyl and $C_1$—$C_4$ alkanoyloxyphenyl were excluded from the original definition of $R_3$. The formula (I) compounds wherein $R_3$ is hydroxyphenyl may be prepared by hydrolysis of either the corresponding $R_3$=methoxyphenyl or benzyloxyphenyl derivatives by conventional procedures, e.g., by treatment with HBr or HI in acetic acid. Acylation of the resultant $R_3$=hydroxyphenyl derivatives by $C_1$—$C_4$ alkanoic acids in the presence of excess dicyclohexylcarbodiimide affords the corresponding $R_3$=$C_1$—$C_4$ alkanoyloxy derivatives of formula (I).

Another method of preparing the formula (I) compounds wherein Z is (a), (b) or (c), Z in such case being designated $Z_2$, utilizes the methodology described by E. Kuhle, *Angew. Chem. Internat. Ed., 8,* 24, 26, (1969) and references cited therein, which involves the sequential displacement of chloride from an appropriate isocyanide dichloride (XII) wherein $R_3$ is other than hydroxyphenyl. The latter, the preparation of which is described by E. Kuhle et al. in *Angew. Chem. Internat. Ed., 6,* 649 (1967), is reacted with the amine, $HNR_1R_2$, in the presence of a trialkylamine, e.g., triethylamine, in a suitable reaction-inert aprotic anhydrous solvent such as an ether, for example, diethyl ether or tetrahydrofuran, a halohydrocarbon or an aromatic hydrocarbon, to give the monochloride compound (XIII) which is separated from triethylamine hydrochloride, by filtration, and then used *in situ* as the filtrate in the reaction with $Z_2H$ (II) to form final product (I).

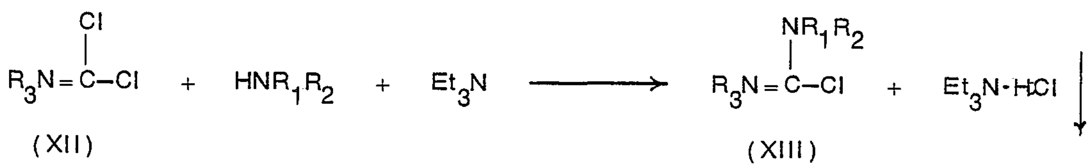

(XII) (XIII)

Preferably, two molar equivalents of $Z_2H$ (II) are then reacted with each molar equivalent of (XIII). Alternatively, when $Z_2$ is equal to the diaza function represented by letter (c), a 1:1 molar equivalent ratio of reactants may be employed in which case a molar equivalent of an appropriate halogen acid scavenger, for example, a base such as triethylamine, is added to pick up the hydrochloric acid released during the course of the reaction. In either route, the resultant precipitated acid addition salt is removed from the reaction mixture, e.g., by filtration, and the desired product (I) which remains *in situ* in the filtrate, is conveniently isolated by conversion to an acid addition salt in the conventional manner.

a. 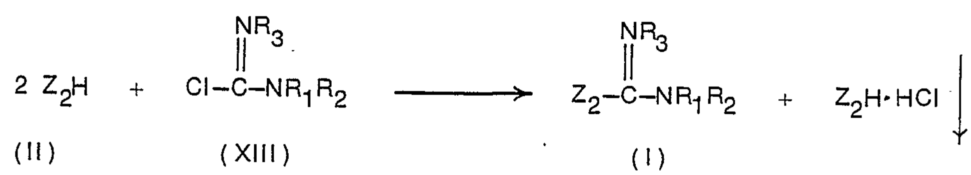

(II) (XIII) (I)

b. 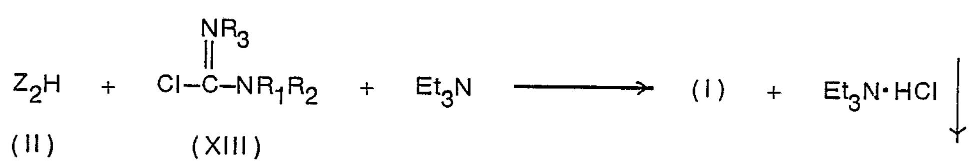

(II) (XIII)

Still another method of preparing the formula (I) compounds utilizes the methodology of H.G. Viehe and Z. Janousek, Angew. Chem. internat. Ed., *12*(10), 806 (1973), from the interaction of an appropriate dichloromethyleneammonium salt of formula (XIV) with an equivalent amount of an appropriate amine of formula (XV), wherein $R_3$ is other than hydroxyphenyl, in the presence of at least two equivalents of an appropriate acid scavenger, e.g., triethylamine, to yield the aforementioned product (XIII) which may then be reacted with $Z_2H$ (II) form end products (I) as previously described.

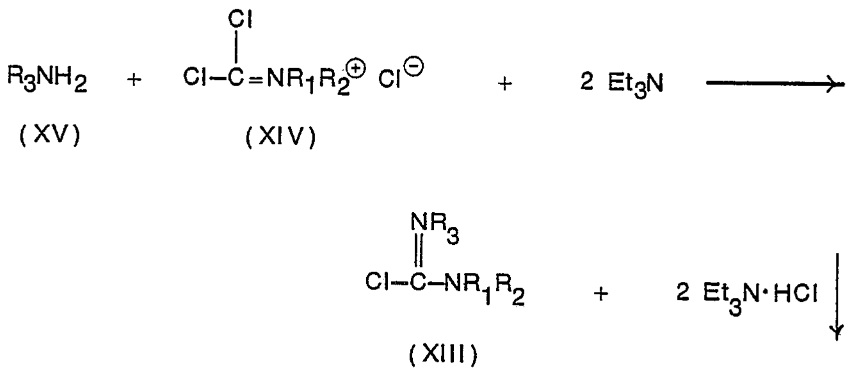

(XV) (XIV)

(XIII)

In each of the foregoing synthetic procedures for preparing formula (I) compounds wherein Z is (a), (b) or (c), Z in such case being designated $Z_2$, hydroxyphenyl was excluded from the original definition of $R_3$. The formula (I) compounds wherein $R_3$ is hydroxyphenyl may be prepared either (i) by debenzylation of the corresponding $R_3$=benzyloxyphenyl derivatives by conventional procedures, e.g., by reduction via hydrogenation in the presence of palladium-on-carbon catalyst or (ii) by hydrolysis of the corresponding $R_3$=benzyloxyphenyl and methoxyphenyl derivatives by conventional procedures, e.g., by treatment with HBr or HI in acetic acid. This latter procedure is preferred when sulfur is present in the molecule, e.g., wherein

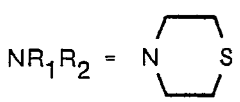

(thiamorpholine)

It is known that sulfur, when present in organic molecules in the divalent state, has a poisoning effect on hydrogenation catalysts, e.g., palladium-on-charcoal. Therefore, it is preferred that compounds of formula (I) wherein $NR_1R_2$=thiamorpholine and $R_3$=hydroxyphenyl, be prepared by hydrolysis of either a corresponding $R_3$=methoxy-Ph or $R_3$=benzyloxy-Ph derivatives of formula (I) by the action of either NHr or HI in acetic acid.

The subject compounds of formula (I) and the acid addition salts thereof possess valuable pharmacological properties, particularly as hypoglycemic agents. Their ability to lower blood sugar can be demonstrated in the following rat glucose tolerance test, which test is a standard and extremely sensitive procedure used in the diagnosis of diabetes and hypoglycemic disease states.

In this test, male Sprague-Dawley rats (Charles River 184—250 grams) are given water *ad libitum* and fasted 24 hours prior to the experiment. Two to five rats are used for each test and control group. Test compounds, 1—200 mg/kg are administered (s.c., i.p. or orally) suspended in 0.5 to 1.0 milliliter, but preferably the former, of 0.5—1.0% methylcellulose vehicle. Control animals are given an equal amount of vehicle. Serial blood samples (0.1 milliliter) are obtained from the tail without anesthesia prior to and at 30, 60, 90, 120, 150 and 180 minutes after administration of 0.8 to 1.0 gram of glucose per kilogram of body weight in 1 milliliter of water. (The glucose is given orally if the test compound has been given parenterally, and subcutaneously if the test compound has been given orally.) Specimens of blood are immediately deproteinized with aqueous solutions of $Ba(OH)_2$ and $ZnSO_4$ and glucose levels are determined using the glucose oxidase assay described by L. P. Cawley et al., "Ultra Micro Chemical Analysis of Blood Glucose with Glucose Oxidase," *Amer. J. Clin. Path., 32,* 195 (1959). The blood glucose values at each time point are expressed in terms of milligram percent (mg glucose/100 ml of blood). The mean glucose values of the controls are compared statistically by the Student' t-Test to the means of the experimental group at each of the corresponding time points. If the compound lowers the blood glucose significantly at any time at a 95% confidence limit, the compound is considered to have hypoglycemic activity. The blood glucose lowering, expressed as percent lowering, is obtained by dividing the difference between the mean blood glucose values for test and control animals by the mean glucose value for the control animal.

In addition to their hypoglycemic activity, certain of the subject compounds have been found to possess antisecretory activity and/or cardiovascular activity as demonstrated in tests described in Belgian Pat. No. 852565.

The subject compounds (I), in base or salt form, may be formulated into conventional liquid and solid pharmaceutical dosage forms and preparations, for example, for oral or parenteral administration, according to standard pharmaceutical techniques in the art.

The following examples are intended to illustrate the scope of the present invention. Unless otherwise stated, all parts are by weight.

Example I

N(1-methyl-2-imidazolidinylidene)-N'-phenylthiourea:

2-Amino-1-methylimidazoline hydroiodide (26.75 g, 0.117 mole) is converted to its free base form by treatment with 50% sodium hydroxide. The base is extracted with methylene chloride and the extract dried over potassium carbonate and filtered. To this solution is added phenylisothiocyanate (15.81 g, 0.117 mole) and the reaction mixture is refluxed for three hours. The hot solution is filtered, concentrated to a small volume *in vacuo*, ether added, chilled, and the solid filtered off to give 23.6 g (86.1%) of N-(1-methyl-2-imidazolidinylidene)-N'-phenylthiourea; m.p. 205—208°C.

Example II

Methyl N-(1-methyl-2-imidazolidinylidene)-N'-phenylcarbamimidothioate hydroiodide:

A mixture of 22.0 g (0.094 mole) of N-(1-methyl-2-imidazolidinylidene)-N'-phenylthiourea and 14.9 g (0.105 mole) of methyliodide in 500 ml of acetone is refluxed for 2.5 hours. The reaction mixture is evaporated *in vacuo* and the oil obtained is crystallized from acetone-ether (1:1) to give 30 g (85%) of methyl N-(1-methyl-2-imidazolidinylidene)-N'-phenylcarbamimidothioate hydroiodide; m.p. 122—126°C.

Example III

The procedures of Examples I and II are followed except that an equivalent amount each of the appropriate reactants of formula (II) and of formula (III) are initially employed to prepare the desired thiourea of formula (IV), which is then treated with an appropriate S-methylating agent to yield as final products the indicated salt of the listed pseudothioureas of formula (V):

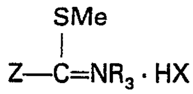

(V)

| No. | Z | $R_3$ | HX |
|---|---|---|---|
| 1 | (c) | 4-F-2-Me-Ph | $HO_3SMe$ |
| 2 | (a) | 3-$CF_3$-Ph | 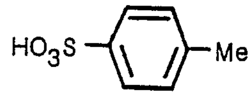 |
| 3 | (b) | 3-Br-Ph | HCl* |
| 4 | (c) | 3,4-diCl-Ph | HI |
| 5 | (b) | 2,3,4-triCl-Ph | $HO_3SF$ |
| 6 | (c) | 5-Cl-2,4-diOMe-Ph | HI |
| 7 | (a) | 4-Br-2-Cl-Ph | $HO_3SMe$ |
| 8 | (a) | 4-I-Ph | HI |
| 9 | (c) | 4-OEt-Ph | HI |
| 10 | (a) | 4-OMe-2-Me-Ph | HI |
| 11 | (b) | 3,4,5-triOMe-Ph | HI |
| 12 | (c) | 4-OBz-Ph | HI |
| 13 | (a) | 2-Et-6-Me-Ph | HI |
| 14 | (c) | 2,4,5-triMe-Ph | HI |
| 15 | (b) | 4-*n*-Bu-Ph | HI |
| 16 | (a) | 4-$NO_2$-Ph | HBr* |
| 17 | (a) | diphenylmethyl | HI |
| 18 | (c) | d,l-$\alpha$-Me-Bz | HI |
| 19 | (a) | $\alpha,\alpha$-diMe-Phenethyl | HI |
| 20 | (c) | 1,2-diphenethyl | HI |
| 21 | (a) | 1-adamantyl | HI |
| 22 | (c) | cyclohexyl | HI |
| 23 | (a) | *endo*-2-norbornyl | HI |
| 24 | (c) | *tert*-butyl | HI |
| 25 | (a) | *tert*-octyl | HI |
| 26 | (b) | 3,4-methylenedioxy-Ph | HI |
| 27 | (b) | Ph | HI |
| 28 | (a) | 4-OBz-Ph | HI |

* When MeBr and MeCl are used as the S-methylating agent, the reaction is preferably run in a sealed vessel.

(*NOTE:* Me = methyl; Et = ethyl; Bu = butyl; Ph = phenyl and Bz = benzyl.)

Example IV

N-(1-methyl-2-imidazolidinylidene)-N′-phenyl-1-pyrrolidinecarboximidamide fumarate:

A mixture of 10.0 g (0.042 mole) of methyl N-(1-methyl-2-imidazolidinylidene)-N′-phenylcarbamimidothioate hydroiodide and 6.4 g (0.090 mole) of pyrrolidine in 200 ml of *t*-butanol is refluxed overnight (about 16 hours) under a slow stream of nitrogen. Sodium hypochlorite and NaOH traps are used to remove the methyl mercaptan formed in the reaction. The reaction mixture is cooled, either is added, and the formed solid is filtered to yield 11.4 g (67%) of hydroiodide salt: m.p. 237—240°C. The conversion to the free base is done by partitioning the hydroiodide between *3N* sodium hydroxide and methylene chloride. The organic layer is dried over potassium carbonate, filtered and evaporated *in vacuo* to give 7.6 g (0.028 mole) of free base, which is converted to the fumarate salt with an equimolar amount of fumaric acid in 2-propanol. Two recrystallizations from ethanol-ether gives 7.5 g (68%) of pure product, N-(1-methyl-2-imidazolidinylidene)-N′-phenyl-1-pyrrolidinecarboximidamide fumarate, m.p. 163——165°C.

Example V

By following the procedure of Example IV, except that an equivalent amount of each pseudothiouronium salt in Example III is reacted with an equivalent amount of an appropriate amine ($HNR_1R_2$), there are obtained as final products, with or without conversion to the indicated acid addition salt, the following compounds of formula (I). In the table, the compound number corresponds to the compound number in the table of Example III.

| No. | $-NR_1R_2$ | Hx |
|---|---|---|
| 1 | 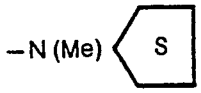 | $HO_3SMe$ |
| 2 | 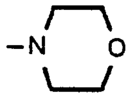 | 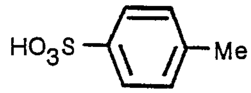 |
| 3 | 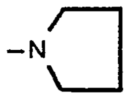 | HCl |
| 4 | 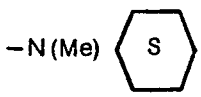 | HI |
| 5 | 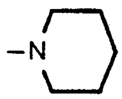 | maleate |
| 6 | 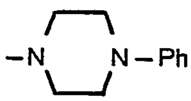 | 2 HI |
| 7 | $-NEt_2$ | $HO_3SMe$ |
| 8 | 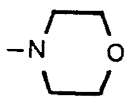 | HI |
| 9 | 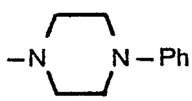 | 2 HI |
| 10 | –N (Me) Bz | HI |
| 11 | 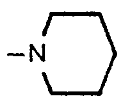 | HI |
| 12 | 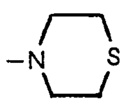 | HI |
| 13 | $-NEt_2$ | fumarate |
| 14 | 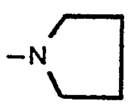 | succinate |
| 15 | 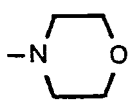 | HI |
| 16 | 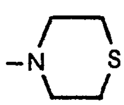 | HBr |
| 17 | 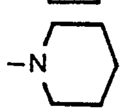 | HI |
| 18 | 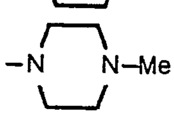 | 2 HI |

| No. | $-NR_1R_2$ | Hx |
|---|---|---|
| 19 | $-NMe_2$ | HI |
| 20 | 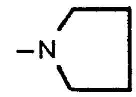 | HI |
| 21 | $-NEt_2$ | HI |
| 22 | 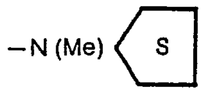 | HI |
| 23 | 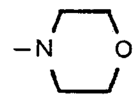 | HI |
| 24 | 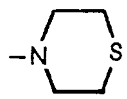 | HI |
| 25 | 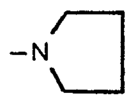 | HI |
| 26 | —N (Me) Bz | HI |
| 27 | 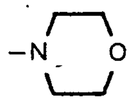 | fumarate |
| 28 | 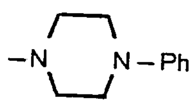 | 2 HI |

Example VI

N-(1,3-dimethyl-2-imidazolidinylidene)-N′-phenyl-1-pyrrolidinecarboximidamide fumarate:

Triethyloxonium fluoroborate is prepared from 1.85 g (0.020 mole) of epichlorohydrin in 7 ml of ether and 3.79 g (0.027 mole) of boron trifluoride in 3 ml of ether. The solid triethyloxonium fluoroborate is dissolved in 15 ml of dry methylene chloride and treated under nitrogen with 1,3-dimethyl-2-imidazolidine-2-one in 25 ml of methylene chloride. The reaction mixture is stirred overnight (about 16 hours) at room temperature under nitrogen. A 25 ml methylene chloride solution of N-phenyl-1-pyrrolidinecarboximidamide free base (obtained from 6.3 g, (0.020 mole) of the corresponding hydroiodide salt with 50% sodium hydroxide and dried over potassium carbonate) is added to the reaction mixture and stirred at room temperature overnight. The solvent is removed *in vacuo* and the oily fluoroborate salt is converted to the free base with 3N sodium hydroxide. The organic layer is dried over potassium carbonate, filtered, evaporated *in vacuo* and the oil converted to the fumarate salt with one equivalent of fumaric acid (1.83 g, 0.0158 mole) in isopropanol to give 6.1 g of impure product.

Recrystallization from a 1:1 mixture of 2-propanol and ether does not purify the product. The fumarate is converted to the free base with 3N sodium hydroxide and it is chromatographed on a dry-packed silica column (eluted with 20% of ammonia-methanol). The column is segmented into 1 inch sections and all the segments containing the pure product (as inciated by thin layer chromatography) are combined and extracted with the above solvent mixture. The solvent is then removed *in vacuo* at room temperature and the solid residue extracted with methanol, dried over molecular sieves (3A); filtered over diatomaceous earth and evaporated *in vacuo*. The residue is taken up in chloroform, dried over potassium carbonate, filtered and evaporated to yield pure free base, N-(1,3-dimethyl-2-imidazolidinylidene)-N′-phenyl-1-pyrrolidinecarboximidamide.

Conversion to the fumarate is done with one equivalent of fumaric acid in 2-propanol. Three recrystallizations from 2-propanol-ethylacetate (1:1) gives the pure fumarate salt as a white solid, m.p. 171.5—173.5°C.

Example VII

The procedure of Example VI is followed, except that an equivalent amount of an appropriate guanidine derivative of formula (VII) is substituted for the one used therein, to yield as final products the following N-(1,3-dimethyl-2-imidazolidylidene) type compounds of formula (I) in the form of the indicated acid addition (HX) salt.

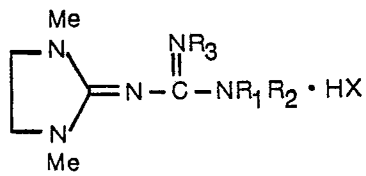

| No. | $R_3$ | $-NR_1R_2$ | HX |
|---|---|---|---|
| 1 | Ph | 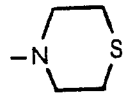 | HI |
| 2 | 4-Me-Ph | 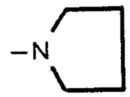 | HCl |
| 3 | 3-Cl-Ph | 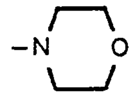 | $H_3PO_4$ |
| 4 | Ph | −N (Me) 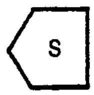 | HI |
| 5 | *exo*-2-norbornyl | 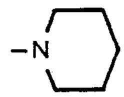 | HBr |
| 6 | *endo*-2-norbornyl | 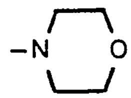 | $MeSO_3H$ |
| 7 | 3,4-diMeO-Ph | $-NEt_2$ | HI |
| 8 | 1-naphthyl | −N (Me) 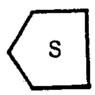 | fumarate |
| 9 | 1-adamantyl | 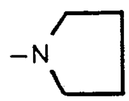 | HI |
| 10 | 2,2-diphenethyl | 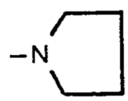 | HCl |
| 11 | cyclopentyl | $-NEt_2$ | benzoate |
| 12 | *tert*-octyl | 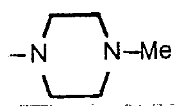 | 2 HI |
| 13 | 4-SMe-Ph | 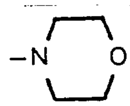 | HCl |
| 14 | $3-CF_3-Ph$ | −N (Me) 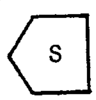 | $HNO_3$ |
| 15 | $4-NO_2-Ph$ | 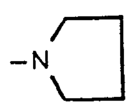 | $H_2SO_4$ |
| 16 | 4-OBz-Ph | $-NEt_2$ | HI |
| 17 | 4-OBz-Ph | 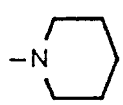 | HI |

Example VIII

N-(1-methyl-1H-imidazol-2-yl)-N'-phenyl-1-pyrrolidinecarboximidamide fumarate:

To a chilled solution of 4.26 g (0.060 mole) of pyrrolidine in 45 ml of anhydrous ether is added dropwise under nitrogen a solution of 7.44 g (0.060 mole) of phenylisocyanide dichloride and 6.06 g (0.060 mole) of triethylamine in 45 ml of anhydrous ether. The ice bath is removed and the white suspension is stirred at room temperature for two hours. The precipitated triethylamine hydrochloride is filtered off and washed with three 30 ml portions of ether. To the combined ether filtrates is added 6.06 g (0.060 mole) of triethylamine and the mixture is again chilled in an ice-bath. A solution of 2-amino-1-methylimidazole (prepared from the corresponding hydrochloride salt with 50% sodium hydroxide and extracted with methylene chloride) in methylene chloride is added dropwise under nitrogen to the reaction mixture. The colorless solution is allowed to come to room temperature and stirred under nitrogen overnight. After evaporation of the solvent, the residue is treated with $CH_2Cl_2$ and converted to the free base form with cold 3N sodium hydroxide. The organic layer is dried over potassium carbonate, filtered and the solvent evaporated *in vacuo* to give 13.8 g (95%) of an oil. Acid-base extraction gives 6.1 g (42%) of the crude free base, N-(1-methyl-1H-imidazol-2-yl)-N'-phenyl-1-pyrrolidinecarboximidamide. Recrystallization from benzene-hexane (1:1) yields pure free base, m.p. 115—118°C, which is converted to the fumarate salt with one equivalent of fumaric acid (2.4 g, 0.002 mole) in isopropanol. Recrystallization from methanol-ether (1:1) affords 7.0 g of the pure salt, N-(1-methyl-1H-imidazol-2-yl)-N'-phenyl-1-pyrrolidinecarboximidamide fumarate, m.p. 156.5—159.5°C.

Example IX

By following the procedure of Example VIII except that an equivalent amount of an appropriate isocyanide dichloride of formula (XII) is used as the reactant with 2-amino-1-methylimidazole, the following N-(1-methyl-1H-imidazol-2-yl) type products of formula (I) are obtained either as the free base or as the indicated acid addition (HX) salt form.

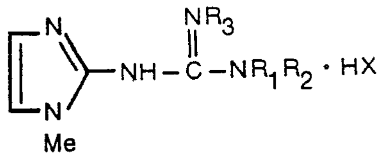

| No. | $R_3$ | $-NR_1R_2$ | HX |
|---|---|---|---|
| 1 | 2-Br-4-Me-Ph | 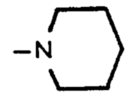 | — |
| 2 | cyclohexyl | 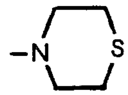 | HCl |
| 3 | 4-Me-Ph | −N(Me) 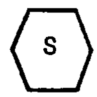 | HCl |
| 4 | 4-Cl-2,6-diEt-Ph | 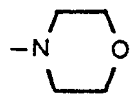 | — |
| 5 | 3-Cl-4-OMe-Ph | 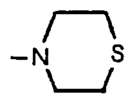 | — |
| 6 | 3-F-Ph | −N(Me) 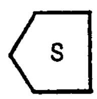 | $HNO_3$ |
| 7 | 3-$CF_3$-Ph | 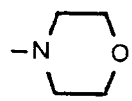 | — |
| 8 | 1-naphthyl | 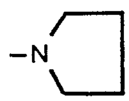 | fumarate |
| 9 | Ph | 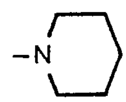 | fumarate |
| 10 | cyclohexyl | 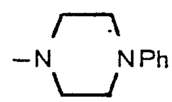 | — |
| 11 | Bz | 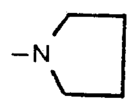 | — |
| 12 | Ph | $-NEt_2$ | HCl |
| 13 | 4-OBz-Ph | 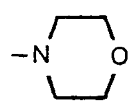 | — |
| 14 | 4-OBz-Ph | −N(Me) 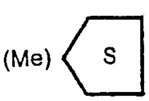 | — |

Example X

N-(1-methylimidazol-2-yl)-N′-phenylthiourea:

A 4.8 g (0.036 mole) sample of 2-amino-1-methylimidazole hydrochloride is suspended in 20 ml of methylene chloride and stirred with 10 ml of 50% NaOH. The organic layer is separated and the aqueous layer is extracted twice with 20 ml portions of fresh methylene chloride. The combined organic

extracts are dried over $K_2CO_3$ and filtered. The filtrate is treated with 4.86 g (0.036 mole) of phenyl isothiocyanate. The solution is heated under reflux for 4 hr. Evaporation of the solvent *in vacuo* followed by recrystallization of the residue from acetone-ether (1:1) and MeOH-ether (1:1) gives the product, N-(1-methylimidazol-2-yl)-N'-phenylthiourea; m.p. 170—172°C.

Example XI

Methyl N-(1-methylimidazol-2-yl)-N'-phenylcarbamimidothioate hydroiodide:

A solution of 3.0 g (0.013 mole) of N-(1-methylimidazol-2-yl)-N'-phenylthiourea in 50 ml of methanol is added 2.13 g (0.015 mole) of methyl iodide. The solution is heated under reflux for 2 hr, the solvent is evaporated and the residue is recrystallized from acetone-ether to give methyl N-(1-methylimidazol-2-yl)-N'-phenylcarbamimidothioate hydroiodide; m.p. 115—118°C.

Example XII

By following the procedure of Example X, except that an equivalent amount of an appropriate isothiocyanate is employed instead of the phenyl isothiocyanate used therein, and by then utilizing an equivalent amount of the thus-obtained thiourea as the precursor to be S-methylated according to the procedure of Example XI, the following pseudothioureas of formula (V) are obtained.

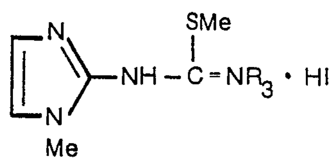

| No. | $R_3$ | No. | $R_3$ |
|---|---|---|---|
| 1 | 2,4-diF-Ph | 8 | 4-Cl-Ph |
| 2 | 4-$CF_3$-Ph | 9 | 3,5-diOMe-Ph |
| 3 | 2,4,5-triCl-Ph | 10 | 3-OBz-Ph |
| 4 | 5-Cl-2-OMe-Ph | 11 | 2,4-diMe-Ph |
| 5 | 4-Br-Ph | 12 | 3-$NO_2$-Ph |
| 6 | 4-Br-3-Cl-Ph | 13 | 2-OMe-5-Me-Ph |
| 7 | 4-OEt-Ph | 14 | 3,4-diCl-Ph |
| | | 15 | 4-OBz-Ph |

Example XIII

N-(1-methylimidazol-2-yl)-N'-phenyl-4-morpholinecarboximidamide hydrochloride:

A solution of 1.5 g (0.004 mole) of methyl N-(1-methylimidazol-2-yl)-N'-phenylcarbamimidothioate hydroiodide and 0.73 g (0.0084 mole) of morpholine in 30 ml of *t*-BuOH is refluxed overnight. Upon cooling, ether is added and morpholine hydroiodide is removed by filtration. The filtrate is taken to dryness and the residue is taken up in $CH_2Cl_2$ and converted to the free base with 3N NaOH. The organic layer is separated, dried over $K_2CO_3$, filtered and the solvent is removed *in vacuo* to give a residue of N-(1-methylimidazol-2-yl)-N'-phenyl-4-morpholine caboximidamide, which is taken up in acetone and converted to the hydrochloride salt with ethereal HCl.

Example XIV

The procedure of Example XIII is followed except that an equivalent amount of each pseudothiourea obtained in Example XII is reacted with an equivalent amount of an appropriate amine of the formula $HNR_1R_2$ to yield the following products of formula (I).

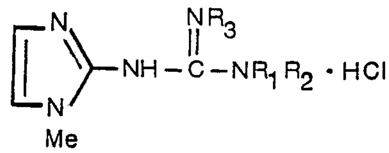

| No. | $R_3$ | $-NR_1R_2$ |
|---|---|---|
| 1 | 2,4-diF-Ph | 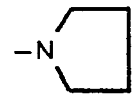 |
| 2 | 4-$CF_3$-Ph | 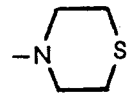 |
| 3 | 2,4,5-triCl-Ph | —N (Me) 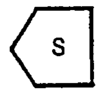 |
| 4 | 5-Cl-2-OMe-Ph | 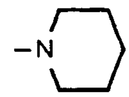 |
| 5 | 4-Br-Ph | —N (Me) 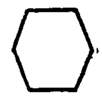 |
| 6 | 4-Br-3-Cl-Ph | $-NEt_2$ |
| 7 | 4-OEt-Ph | 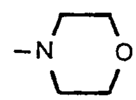 |
| 8 | 4-Cl-Ph | 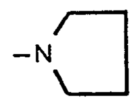 |
| 9 | 3,5-diOMe-Ph | 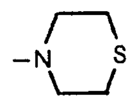 |
| 10 | 3-OBz-Ph | —N (Me) Bz |
| 11 | 2,4-diMe-Ph | 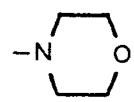 |
| 12 | 3-$NO_2$-Ph | 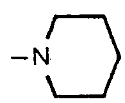 |
| 13 | 2-OMe-5-Me-Ph | —N (Me) Et |
| 14 | 3,4-diCl-Ph | 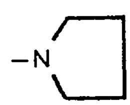 |
| 15 | 4-OBz-Ph | 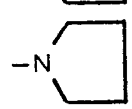 |

Example XV

N-(1-adamantyl)-N'-(1-methylimidazol-2-yl)-1-pyrrolidinecarboximidamide:

To 18.85 g (0.1 mole) of dichloromethylenetetramethylene ammonium chloride in 100 ml of dry methylene chloride is added dropwise a solution of 15.13 g (0.1 mole) of 1-adamantylamine and 20.2

g (0.2 mole) of triethylamine in dry $CH_2Cl_2$ with cooling (ice bath) under dry $N_2$. After the addition is complete, the mixture is stirred at room temperature for 3 hrs. Then a dry methylene chloride solution containing 9.71 g (0.1 mole) of 2-amino-1-methylimidazole and 10.1 g (0.1 mole) of triethylamine is added dropwise over 1 hr with cooling. After the addition the mixture is stirred under dry $N_2$ overnight at room temperature. The mixture is then shaken with excess 3N NaOH and the organic layer is separated, dried ($K_2CO_3$), filtered and the solvent and residual triethylamine is removed *in vacuo* giving N-(1-adamantyl)-N′-(1-methylimidazol-2-yl)-1-pyrrolidinecarboximidamide free base.

Example XVI

By repeating the procedure of Example XV but substituting for dichloromethylenetetramethyleneammonium chloride an appropriate dichloromethyleneammonium salt and an appropriate $R_3NH_2$ amine for 1-adamantylamine, the following N-(1-methylimidazol-2-yl)-N′-substituted-1- (or 4) carboximidamines are obtained. Treatment with appropriate acid (HX) affords the indicated acid addition salt.

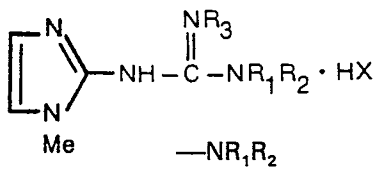

| No. | $R_3$ | $—NR_1R_2$ | HX |
|---|---|---|---|
| 1 | cyclohexyl | 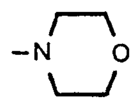 | HI |
| 2 | *α,α*-diMe-phenethyl | $-NEt_2$ | $HO_3SMe$ |
| 3 | d,l-*α*-Me-Bz | $-NMe_2$ | HCl |
| 4 | *exo*-2-norbornyl | 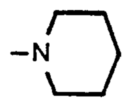 | — |
| 5 | benzhydryl | 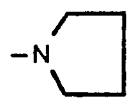 | — |
| 6 | 2,2-diphenylethyl | 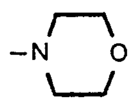 | *p*-TsOH |
| 7 | 3,4-diOMe-Ph | −N (Me) Bz | — |
| 8 | Bz | $-N(IsoPr)_2$ | $H_2SO_4$ |
| 9 | 3-Cl-Ph | −N (Me) 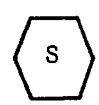 | $H_3PO_4$ |
| 10 | 2,4-diMe-Ph | 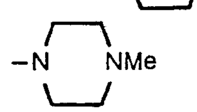 | L(+)-tartrate |
| 11 | 1,2-diphenylethyl | $-NMe_2$ | — |
| 12 | 4-OBz-Ph | 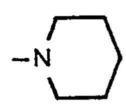 | HCl |
| 13 | neopentyl | 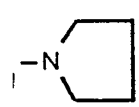 | — |
| 14 | *tert*-octyl | 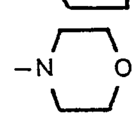 | — |

### Example XVII

N-(4,5-dihydro-1-methylimidazol-2-yl)-N′-(4-hydroxyphenyl)-1-pyrrolidinecarboximidamide hydroiodide:

To a solution of 37.8 g (0.1 mole) of N-(4-benzyloxyphenyl)-N′-4,5-dihydro-1-methylimidazol-2-yl-1-pyrrolidinecarboximidamide in 150 ml of glacial acetic acid in a 500 ml hydrogenation bottle, is added 0.5 g of 30% Pd/C catalyst. The mixture is hydrogenated at a starting pressure of 60 p.s.i. for 4 hr, then filtered from catalyst and the acetic acid removed *in vacuo*. The residue is taken up in *tert*-butanol and treated with a stoichiometric amount of 48% HI. Ether is added causing N-(4,5-dihydro-1-methylimidazol-2-yl)-N′-(4-hydroxyphenyl)-1-pyrrolidinecarboximidamide hydroiodide to precipitate.

### Example XVIII

The debenzylation procedure of Example XVII is followed, except that an equivalent amount of an appropriate $R_3$=benzyloxyphenyl precursor is initially employed, to yield the following $R_3$=hydroxyphenyl derivatives of formula (I) in the form of the hydroiodide salt.

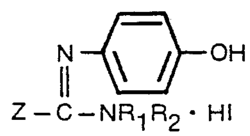

| Z | $-NR_1R_2$ |
|---|---|
| 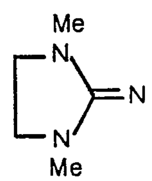 | $-NEt_2$ |
| (do) | 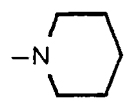 |
| 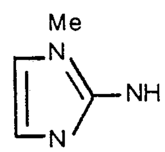 | 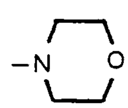 |
| (do) | 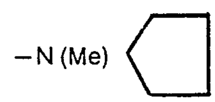 |
| 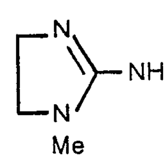 | 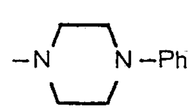 |

### Example XIX

2-Imino-1,3-dimethylimidazolidine sulfate (2:1 salt):

Triethyloxonium tetrafluoroborate is prepared in ether on a 1.2 mole scale from 327.1 g (1.6 mole) of boron trifluoride etherate and 111.0 g (1.2 mole) of epichlorohydrin under dry $N_2$. The resulting crystals are washed with fresh anhydrous ether by decantation (2 × 250 ml). The resulting crystals of triethyloxonium tetrafluoroborate are dissolved in 1 liter of dry methylene chloride. To this solution is added 114 g (1 mole) of 1,3-dimethylimidazolidine-2-one. The mixture is allowed to stir at ambient temperatures for 3 hrs. Then anhydrous $NH_3$ is added vigorously for 10 min. The mixture becomes warm. Ammonia addition is continued at a gentle rate for 20 min more and then allowed to stir for 0.5 hr. The mixture is filtered from inorganic material, concentrated to a volume of 200 ml and let stand overnight. Treatment with 100 ml of 50% NaOH with cooling (ice-water) and shaking converts the product to free base. The aqueous layer is washed with fresh $CH_2Cl_2$ (3 × 100 ml). The combined, dried

($K_2CO_3$) organic layers are filtered (diatomaceous earth) giving a cloudy filtrate. Removal of solvent *in vacuo* with gentle heat causes separation of more solid. To the oily suspension is added 200 ml ether and the solution is refiltered. The filtrate is reduced to a volume of about 100 ml and then taken up in MeOH (200 ml). The resulting solution is cooled while concentrated $H_2SO_4$ is added until pH 6. Boiling on a stirring hot place with addition of 2-PrOH to replace lost MeOH gives a crop of solid which separates from the hot solution. The crystals are filtered and the filtrate is concentrated with more 2-PrOH added until MeOH is removed. Cooling affords a second crop coated by a syrup. The gummy crystals are washed with fresh 2-PrOH and combined with the first crop. The combined crops are recrysallized by dissolving in MeOH, filtering (diatomaceous earth), adding 2-PrOH and concentrating as before to give pure 2-imino-1,3-dimethylimidazolidine 1/2 sulfate; m.p. >300°C. A second crop is obtained from the mother liquors; m.p. 300°C. Yield 69.5 g total (42.8%).

Example XX

N-(4-Hydroxyphenyl)-N′-(1-methylimidazol-2-yl)-4-thiamorpholinecarboximidamide hydroiodide:

A 50 ml round-bottomed flask is charged with 3.75 g (0.007 mole) of N-(4-benzyloxyphenyl-N′-(1-methylimidazole-2-yl)-4-thiamorpholinecarboximidamide hydroiodide (Ex. V-12) 5.9 g (0.021 mole) of 50% hydriodic acid, and 6.0 g (0.1 mole) of glacial acetic acid. The mixture is heated under reflux for 6 hr, then the excess HI and solvent are removed under reduced pressure leaving as the residue, N-(4-hydroxyphenyl)-N′-(1-methylimidazol-2-yl)-4-thiamorpholinecarboximidamide hydroiodide.

Example XXI

N-(1,2-Dihydro-1-methyl-2(1H)pyridinylidene)-N′-phenyl-1-pyrrolidinecarboximidamide fumarate:

A. Triethyloxonium fluoroborate is prepared from 3.70 g (0.040 mole) of epichlorohydrin in 14 ml of ether and 7.58 g (0.054 mole) of boron trifluoride ethereate in 6 ml of ether. The resulting solid triethyloxonium tetrafluoroborate is dissolved in 20 ml of dry methylene chloride and treated under nitrogen with 4.36 g (0.040 mole) of 1-methyl-2-pyridone in 15 ml of methylene chloride. The reaction mixture is stirred under nitrogen at room temperature overnight (about 16 hours). The solvent is evaporated at room temperature, *in vacuo* and the quaternary salt obtained is recrystallized from methanol-ether (1:1) to give 8.0 g (71%) of 2-ethoxy-1-methylpyridinium fluoroborate, m.p. 58—60°C.

B. A mixture of 8.00 g (0.035 mole) of the above pyridinium salt and N-phenyl-1-pyrrolidine-carboximidamide free base (obtained from 11.10 g (0.035 mole) of the corresponding hydroiodide with 3*N*-sodium hydroxide, extracted in methylene chloride, dried over $K_2CO_3$, and the solvent evaporated *in vacuo*, at room temperature) in 50 ml of *t*-butanol is refluxed overnight. The reaction mixture is cooled, ether added, and the solid formed is filtered to give 10.3 g (80%) of N-(1,2-dihydro-1-methyl-2(1*H*)-pyridinylidene)-N′-phenyl-1-pyrrolidinecarboximidamide $HBF_4$, m.p. 184—187°C. Recrystallization from methanol-ether gives the pure compound (by thin layer chromatography). The $HBF_4$ salt is partitioned between 3N sodium hydroxide/methylene chloride. The organic layer is dried over potassium carbonate, filtered and evaporated *in vacuo* to give the base, N-(1,2-dihydro-1-methyl-2(1*H*)pyrridinylidene)-N′-phenyl-1-pyrrolidinecarboximidamide, m.p. 145—146.5°C.

Conversion of the free base to the fumarate salt is accomplished with an equivalent of fumaric acid in 2-propanol. Recrystallization from 2-propanol-ether (some methanol is added to the 2-propanol suspension to dissolve the salt and then boiled off) gives 9.6 g of N-(1,2-dihydro-1-methyl-2(1*H*)pyridinylidene)-N′-phenyl-1-pyrrolidinecarboximidamide fumarate, m.p. 183—185.5°C.

Example XXII

By reacting an equivalent amount of an appropriate guanidine of formula (VII) with the pyridinium salt, 2-ethoxy-1-methylpyridinium fluoroborate, according to the procedure of Example XXI the following respective products of formula (I) are obtained, as the free base or by conversion to the indicated acid addition (HX) salt:

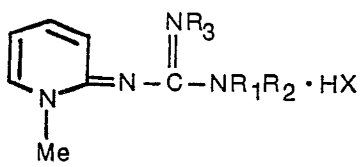

| No. | $R_3$ | $-NR_1R_2$ | HX |
|---|---|---|---|
| 1 | 4-F-2-Me-Ph | 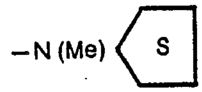 | fumarate |
| 2 | 3-Br-Ph | 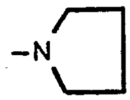 | HCl |
| 3 | 2,3,4-triCl-Ph | 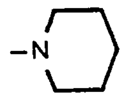 | maleate |
| 4 | 4-Br-2-Cl-Ph | $-NEt_2$ | $HO_3SMe$ |
| 5 | 4-OEt-Ph | 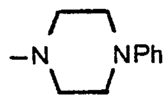 | -base- |
| 6 | 3,4,5-triOMe-Ph | 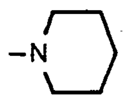 | HI |
| 7 | 2-Et-6-Me-Ph | $-NEt_2$ | fumarate |
| 8 | 4-*n*-Bu-Ph | 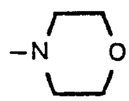 | -base- |
| 9 | diphenylmethyl | 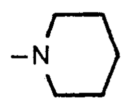 | HCl |
| 10 | α,α-diMe-phenethyl | $-NMe_2$ | -base- |
| 11 | 1-adamantyl | $-NEt_2$ | succinate |
| 12 | *endo*-2-norbornyl | 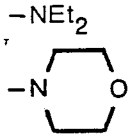 | fumarate |
| 13 | *tert*-octyl | 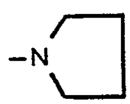 | -base- |
| 14 | Ph | 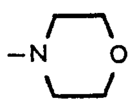 | fumarate |
| 15 | 4-OBz-Ph | $-NEt_2$ | -base- |
| 16 | *exo*-2-norbornyl | 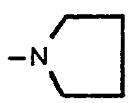 | HCl |
| 17 | 4-SMe-Ph | 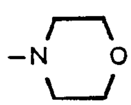 | benzoate |
| 18 | cyclopentyl | $-NEt_2$ | HBr |
| 19 | 1-naphthyl | 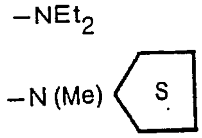 | HBr |

| No. | $R_3$ | $—NH_1R_2$ | HX |
|---|---|---|---|
| 20 | 2,2-diphenethyl | 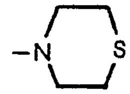 | -base- |
| 21 | Bz | $-NEt_2$ | HCl |
| 22 | 4-OMe-Ph | $-NEt_2$ | HCl |

Example XXIII

N-(1,2-Dihydro-1-methyl-2(1*H*)-quinolinylidene-N'-phenyl-1-pyrrolidinecarboximidamide fumarate (1:1.5):

A. Triethyloxonium fluoroborate is prepared from 3.70 g (0.040 mole) of epichlorohydrin in 14 ml of ether and 7.58 g (0.054 mole) of boron trifluoride etherate in 6 ml of ether. The resulting solid triethyloxonium fluoroborate is dissolved in 20 ml of dry methylene chloride and treated under nitrogen with 6.47 g (0.040 mole) of N-methyl-2-quinolone in 15 ml of methylene chloride. The reaction mixture is stirred under nitrogen at room temperature overnight. The solid is filtered to give 9.2 g (83.5%) of 2-ethoxy-1-methylquinolinium fluoroborate.

B. A mixture of 8.50 g (0.031 mole) of the above quinolinium salt and *N*-phenyl-1-pyrrolidinecarboximidamide in 150 ml of *t*-butanol is refluxed for 24 hours. The reaction mixture is evaporated *in vacuo* and the gummy residue covered with methanol and chilled. The resulting solid is filtered to give 11.2 g (86.8%) of N-(1,2-dihydro-1-methyl-2(1H)-quinolinylidene-N'-phenyl-1-pyrrolidinecarboximidamide $HBF_4$, m.p. 161.5—164°C. Recrystallization from methanol-ether yields the pure $HBF_4$ salt (by TLC), 9.0 g, m.p. 166—169°C. The $HBF_4$ salt is partitioned between 3N-sodium hydroxide and methylene chloride. The organic layer is dried over potassium carbonate, filtered, and evaporated *in vacuo* to give the N-(1,2-dihydro-1-methyl-2(1*H*)-quinolinylidene-N'-phenyl-1-pyrrolidinecarboximidamide as a yellow solid, 6.7 g (80.6%). Conversion of the free base to the fumarate is accomplished with one equivalent of fumaric acid (2.9 g, 0.025 mole) in 2-propanol. Two recrystallizatons from 2-propanol-ether give 7.5 g of pure *N*-(1,2-dihydro-1-methyl-2(1*H*)-quinolinylidene)-N'-phenyl-1-pyrrolidinecarboximidamide fumarate (1:1.5), m.p. 209—211°C (dec.).

Example XXIV

By following the procedure of Example XXIII except that an equivalent amount o an appropriate guanidine of formula (III) is reacted with the quinolinium salt, 2-ethoxy-1-methylquinolinium fluoroborate, the following respective products of formula (I) are obtained, as the free base or by conversion to the indicated acid addition (HX) salt:

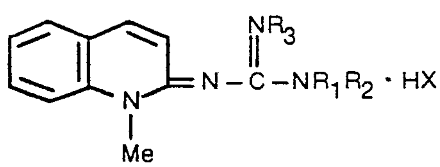

| No. | $R_3$ | $—NR_1R_2$ | HX |
|---|---|---|---|
| 1 | 3-$CF_3$-Ph | 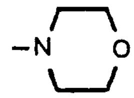 | 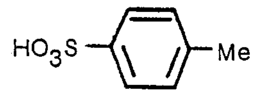 |
| 2 | 3,4-diCl-Ph | 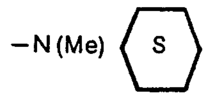 | HBr |
| 3 | 5-Cl-2,4-diOMe-Ph | 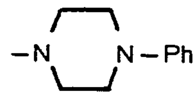 | HCl |
| 4 | 4-I-Ph | 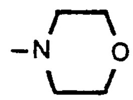 | -base- |
| 5 | 4-OMe-2-Me-Ph | —N (Me) Bz. | -base- |
| 6 | 4-OBz-Ph | 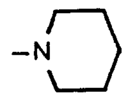 | fumarate |
| 7 | 2,4,5-triMe-Ph | 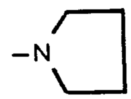 | succinate |
| 8 | 4-$NO_2$-Ph | 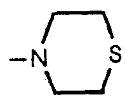 | HCl |
| 9 | d,l-$\alpha$-Me-Bz | 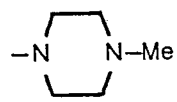 | -base- |
| 10 | 1,2-diphenethyl | 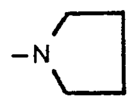 | HI |
| 11 | cyclohexyl | 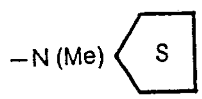 | fumarate |
| 12 | *tert*-butyl | 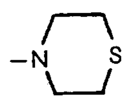 | HCl |
| 13 | 3,4-methylenedioxy-Ph | —N (Me) Bz | fumarate |
| 14 | 4-OBz-Ph | 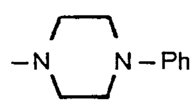 | 2 HI |
| 15 | Ph | $—NEt_2$ | HCl |
| 16 | 1-adamantyl | $—NEt_2$ | HCl |
| 17 | d,l-$\alpha$-Me-Bz | $—NMe_2$ | HBr |
| 18 | *exo*-2-norbornyl | 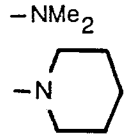 | -base- |
| 19 | benzhydryl | 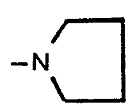 | -base- |

| No. | $R_3$ | $-NR_1R_2$ | HX |
|---|---|---|---|
| 20 | Bz | $-N(IsoPr)_2$ | $H_2SO_4$ |
| 21 | neopentyl | 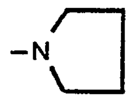 | HCl |
| 22 | 4-OMe-Ph | $-NEt_2$ | HCl |

Example XXV

N′-(4-Methoxyphenyl)-N-(1,2-dihydro-1-methyl-2(1H)-pyridinylidene)-1-piperidinecarboximidamide hydroiodide:

A 100 ml round-bottomed flask is charged with 0.0126 mole of N-(4-methoxyphenyl)-1-piperidinecarboximidamide, 5.2 g (0.037 mole) of anhydrous potassium carbonate, 3.39 g (0.014 mole) of 2-ethoxy-1-methylpyridinium fluoborate and 20 ml of *t*-BuOH. The mixture is allowed to reflux overnight. After filtration, the solvent is removed *in vacuo,* and the residue taken up in methylene chloride and shaken with cold 20% NaOH. The organic layer is dried ($K_2CO_3$), filtered and the solvent removed *in vacuo*. Conversion to the HI salt in ether solution, affords N′-(4-methoxyphenyl)-N-(1,2-dihydro-1-methyl-2(1H)-pyridinylidene)-1-piperidinecarboximidamide hydroiodide.

Example XXVI

N′-(4-Hydroxyphenyl-N-(1,2-dihydro-1-methyl-2(1H)-pyridinylidene)-1-piperidinecarboximidamide monohydroiodide:

A 50 ml round-bottomed flask is charged with 0.00690 mole of N′-(4-methoxyphenyl-N-(1,2-dihydro-1-methyl-2(1H)-pyridinylidene)-1-piperidinecarboximidamide hydroiodide, 5.89 g (0.021 mole) of 50% HI and 6.0 g of glacial acetic acid. This mixture is heated under reflux for 6 hr. Evaporation of the solvent and excess HI *in vacuo* gives N′-(4-hydroxyphenyl-N-(1,2-dihydro-1-methyl-2(1H)-pyridinylidene)-1-piperidinecarboximidamide monohydroiodide.

Example XXVII

N-(4-methoxyphenyl)-N′-(1,2-dihydro-1-methyl-2(1H)-quinolinylidene)-1-piperidinecarboximidamide:

A mixture of 0.010 mole of N-(4-methoxyphenyl)-1-piperidinecarboximidamide, 2.89 g (0.011 mole) of 2-ethoxy-1-methylquinolinium tetrafluoroborate, 4.14 g (0.030 mole) of $K_2CO_3$ and 20 ml of *t*-BuOH is allowed to reflux overnight protected by a $CaCl_2$ drying tube. The reaction mixture is filtered and the solvents evaporated *in vacuo.* The residue is treated with cold 20% NaOH and extracted with $CH_2Cl_2$. The combined organic layers are dried ($K_2CO_3$), filtered and evaporated *in vacuo* to give N′-4-methoxyphenyl)-N-(1,2-dihydro-1-methyl-2(1H)-quinolinylidene)-1-piperidinecarboximidamide in the free base form.

Example XXVIII

N-(4-hydroxyphenyl)-N′-(1,2-dihydro-1-methyl-2(1H)-quinolinylidene-1-piperidinecarboximidamide hydroiodide:

A mixture of 0.01 mole of N-(4-methoxy-N′-(1,2-dihydro-1-methyl-2(1*H*)-quinolinylidene-1-piperidinecarboximidamide, 7.57 g (0.03 mole) of 50% HI, and 7.0 g of glacial acetic acid is heated under reflux for 6 hr. Solvent and excess HI are removed *in vacuo* to give N-(4-hydroxyphenyl)-N′-(1,2-dihydro-1-methyl-2(1H)-quinolinylidene-1-piperidinecarboximidamide hydroiodide.

Example XXIX

N-(4-Acetoxyphenyl-N′-(1,2-dihydro-1-methyl-2(1H)-quinolinylidene-1-piperidinecarboximidamide hydroiodide:

A solution of 0.01 mole of the compound of Example XXVIII in 10 ml of glacial HOAc and 20 ml of methylene chloride is treated with 20.6 g (0.1 mole) of dicyclohexylcarbodiimide under argon. After stirring at ambient temperatures overnight, the formed dicyclohexylurea is removed by filtration and the filtrate is taken to dryness *in vacuo.* The residue is triturated with anhydrous ether (3 x 100 ml) to remove unchanged dicyclohexylcarbodiimide, leaving as the residue, N-(4-acetoxyphenyl)-N′-(1,2-dihydro-1-methyl-2(1H)-quinolinylidene-1-piperidinecarboximidamide hydroiodide.

Example XXX

N-(4-acetoxyphenyl)-N′-(1,2-dihydro-1-methyl-2(1H)-pyridinylidene-1-piperidinecarboximidamide hydroiodide:

A solution of 0.01 mole of the compound of Example XXVI in 10 ml of glacial acetic acid and 20

ml of methylene chloride is treated with 20.6 g (0.1 mole) of dicyclohexylcarbodiimide under argon and the solution is allowed to stir overnight. Workup according to the procedure of Example VI affords as the residue, N-(4-acetoxyphenyl)-N'-(1,2-dihydro-1-methyl-2(1H)-pyridinylidene-1-piperidinecarboximidamide hydroiodide.

### Example XXXI

N-(3-Methyl-2-thiazolidinylidene)-*N'*-phenylthiourea:

A solution of 5.9 g (0.043 mole) of 2-imino-3-methylthiazolidine and an appropriate amount of phenylisothiocyanate in 70 ml of dry benzene is refluxed under nitrogen for 2.5 hrs. Some ether is added to the cooled reaction mixture and solids are filtered off, 9.8 g (90%). Recrystallization from acetonitrile-ether (1:1) gives 8.0 g (75%) of pure *N*-(3-methyl-2-thiazolidinylidene)-*N'*-phenylthiourea; m.p. 168.5—170.5°C.

### Example XXXII

By repeating the procedure of Example XXXI but substituting an equivalent amount of an appropriate $R_3NCS$ for the phenylisothiocyanate used therein, there are obtained the following respective N-[2-(3-methyl)-thiazolidinylidene-N'-$R_3$ thiopureas of formula (IV), Z being (f) in each case.

| No. | $R_3$ | No. | $R_3$ |
|---|---|---|---|
| 1 | 3-F-Ph | 21 | 2,4-diMe-Ph |
| 2 | 2,4-diF-Ph | 22 | 2,4,5-triMe-Ph |
| 3 | 4-$CF_3$-PH | 23 | 3-Et-Ph |
| 4 | 2-Cl-5-$CF_3$-Ph | 24 | 4-IaoPr-Ph |
| 5 | 4-Cl-Ph | 25 | 4-*t*-Bu-Ph |
| 6 | 2,4-diCl-Ph | 26 | 3-$NC_2$-Ph |
| 7 | 2,4,5-triCl-Ph | 27 | 3-SMe-Ph |
| 8 | 5-Cl-2-OMe-Ph | 28 | 1-naphthyl |
| 9 | 3-Cl-4-Me-Ph | 29 | benzhydryl |
| 10 | 4-Br-Ph | 30 | d,1-$\alpha$-Me-Bz |
| 11 | 4-Br-3-Cl-Ph | 31 | $\alpha,\alpha$-dimethylphenethyl |
| 12 | 4-Br-3-Me-Ph | 32 | 2,2-diphenylethyl |
| 13 | 3-I-Ph | 33 | 1-adamantyl |
| 14 | 4-OMe-Ph | 34 | cyclopentyl |
| 15 | 2-OMe-5-Me-Ph | 35 | cyclohexyl |
| 16 | 3,5-diOMe-Ph | 36 | *exo*-2-norbornyl |
| 17 | 3,4-methylenedioxy-Ph | 37 | *endo*-2-norbornyl |
| 18 | 4-OEt-Ph | 38 | neopentyl |
| 19 | 3-OBz-Ph | 39 | *tert*-octyl |
| 20 | 3-Me-Ph | 40 | 3,4,5-triOme-Ph |

Note: Me=methyl; Et=ethyl; IsoPr=isopropyl; Bu=butyl; Ph=phenyl; Bz=benzyl.

Example XXXIII

Methyl N-(3-methyl-2-thiazolidinylidene)-N'-phenylcarbamimidothioate hydroiodide:

A suspension of 17.0 g (0.067 mole) of N-(3-methyl-2-thiazolidinylidene)-N'-phenylthiourea and 11.1 g (0.078 mole) of iodimethane in 350 ml of acetone is refluxed for one hour. The solution is cooled at room temperature overnight (about 16 hours) and solids are filtered off, 25.1 g (95.2%), m.p. 159.5—161.5°C. Recrystallization from methanol-ether (1:1) gives pure methyl N-(3-methyl-2-thiazolidinylidene)-N'-phenylcarbamimidothioate; m.p. 163.5—165°C.

Example XXXIV

By repeating the S-methylation procedure of Example XXXIII with each of the thioureas of Example XXXII, the respective corresponding methyl carbamimidothiate hydroiodide salts of formula (V) are obtained (Z being (f) in each case).

Example XXXV

*N*-(3-Methyl-2-thiazolidinylidene)-*N*'-phenyl-1-pyrrolidinecarboximidamide hydroiodide:

A mixture of 11.9 g (0.030 mole) of N-(1-methyl-2-thiazolidinylidene)-N'-phenylcarbamimidothioate hydroiodide and 4.4 g (0.062 mole) of pyrrolidine in 200 ml of *t*-butanol is refluxed for 24 hrs under a slow stream of nitrogen. Sodium hypochlorite and NaOH traps are used to remove the methyl mercaptan formed during the reaction. The reaction mixture is then cooled, ether added, and the solids formed are filtered off to give 9.3 g (75%) of product. Recrystallizations from acetone-ethyl acetate (1:1) gives 7.8 g (62%) of pure N-(3-methyl-2-thiazolidinylidene)-N'-phenyl-1-pyrrolidinecarboximidamide hydroiodide; m.p. 157.5—160°C.

Example XXXVI

N-(3-Methyl-2-thiazolidinylidene)-N-phenyl-4-morpholinecarboximidamide hydroiodide:

The procedure of Example XXXV is repeated except that an equivalent amount of morpholine is substituted for the pyrrolidine used therein to yield the product, N-(3-methyl-2-thiazolidinylidene)-N'-phenyl-4-morpholinecarboximide hydroiodide, m.p. (188°) 190—191.5°C.

Example XXXVII

N-(4-Methoxyphenyl)-N'-(3-methyl-2-thiazolidinylidene)-1-piperidinecarboximidamide fumarate:

The procedure of Example XXXV is repeated except that an equivalent amount of piperidine is substituted for the pyrrolidine and an equivalent amount of the pseudothiouronium salt of Example XXXIV (Compound No. 14) are utilized as reactants. Basification of the resultant HI salt with aqueous NaOH followed by treatment of the base with an equivalent amount of fumaric acid yields the product, N-(4-methoxyphenyl)-N'-(3-methyl-2-thiazolidinylidene)-1-piperidinecarboximidamide fumarate, m.p. 159.5—160°C.

Example XXXVIII

By repeating the procedure of Example XXXV but employing an equivalent amount of an appropriate $R_1R_2NH$ amine and an equivalent amount of the appropriate pseudothiourea from Example XXXIV as starting materials, the following respective compounds of formula (I) are obtained as the hydroiodide salt.

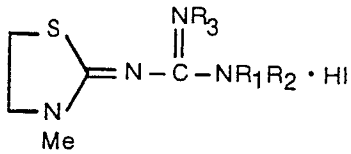

| No. | $R_3$ | $-NR_1R_2$ | No. | $R_3$ | $-NR_1R_2$ |
|---|---|---|---|---|---|
| 1 | 3-F-Ph | 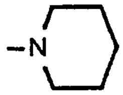 | 21 | 2,4-diMe-Ph | 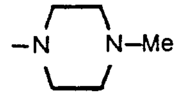 |
| 2 | 2,4-diF-Ph | 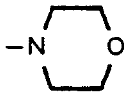 | 22 | 2,4,5-triMe-Ph | 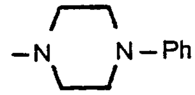 |
| 3 | 4-$CF_3$-Ph | 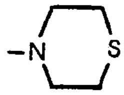 | 23 | 3-Et-Ph | 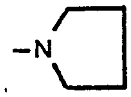 |
| 4 | 2-Cl-5-$CF_3$-Ph | $-NEt_2$ | 24 | 4-IsoPr-Ph | N (Me) Bz |
| 5 | 4-Cl-Ph | $-NMe_2$ | 25 | 4-*t*-Bu-Ph | 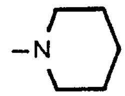 |
| 6 | 2,4-diCl-Ph | −N (Me) Et | 26 | 3-$NO_2$-Ph | 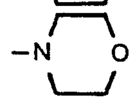 |
| 7 | 2,4,5-triCl-Ph | −N (Me) 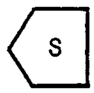 | 27 | 3-SMe-Ph | 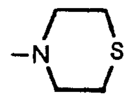 |
| 8 | 5-Cl-2-OMe-Ph | −N (Me) 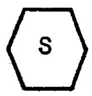 | 28 | 1-naphthyl | $N-Et_2$ |
| 9 | 3-Cl-4-Me-Ph | 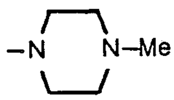 | 29 | benzhydryl | $NMe_2$ |
| 10 | 4-Br-Ph | 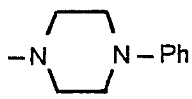 | 30 | d,l-α-Me-Br | N (Me) Et |
| 11 | 4-Br-3-Cl-Ph | 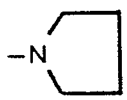 | 31 | α,α-diMe-phenethyl | −N (Me) 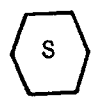 |
| 12 | 4-Br-3-Me-Ph | $-N(Me)CH_2Ph$ | 32 | 2,2-diphenylethyl | −N (Me) 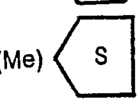 |
| 13 | 3-I-Ph | 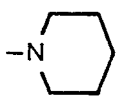 | 33 | 1-adamantyl | 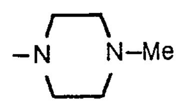 |
| 14 | 4-OMe-Ph | 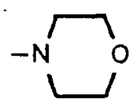 | 34 | cyclopentyl | 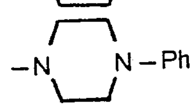 |
| 15 | 2-OMe-5-Me-Ph | 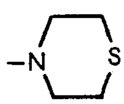 | 35 | cyclohexyl | 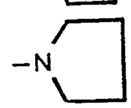 |
| 16 | 3,5-diOMe-Ph | $-NEt_2$ | 36 | *exo*-2-norbornyl | 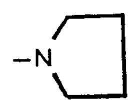 |
| 17 | 3,4-methylene-dioxy-Ph | $-NMe_2$ | 37 | *endo*-2-norbornyl | 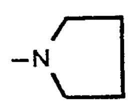 |

| No. | $R_3$ | $—NR_1R_2$ | No. | $R_3$ · | $—NR_1R_2$ |
|---|---|---|---|---|---|
| 18 | 4-OEt-Ph | N (Me) Et | 38 | neopentyl | 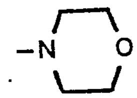 |
| 19 | 3-OBz-Ph | —N (Me)- 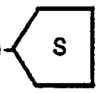 | 39 | *tert*-octyl | 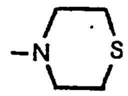 |
| 20 | 3-Me-Ph | —N (Me)- 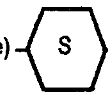 | 40 | 3,4,5-triOMe-Ph | N (Me) $CH_2Ph$ |

## Example XXXIX

*N*-(3-Methyl-2-oxazolidinylidene)-*N'*-phenyl-1-pyrrolidine-carboximidamide fumarate:

Triethyloxonium fluoroborate is prepared from 1.85 g (0.027 mole) of epichlorohydrin in 7 ml of ether and 3.74 g (0.027 mole) of boron trifluoride etherate in 3 ml of ether. The solid triethyloxonium fluoroborate is dissolved in 10 ml of dry methylene chloride and treated under nitrogen with 2.02 g (0.020 mole) of 3-methyloxazolidin-2-one in 20 ml of methylene chloride. The reaction mixture is stirred under nitrogen at room temperature overnight. A 50 ml methylene chloride solution of 4.51 g (0.020 mole) of N-phenyl-1-pyrrolidinecarboximidamide free base (obtained from the corresponding hydrochloride salt with 50% sodium hydroxide and dried over potassium carbonate) is added to the reaction mixture and stirred at room temperature overnight.

The solvent is removed *in vacuo* and the resulting $HBF_4$ salt is recrystallized from 2-propanol/ether (1:1) to give 4.65 g of N-(3-methyl-2-oxazolidinylidene)-N'-phenyl-1-pyrrolidinecarboximidamide ($HBF_4$ as a white solid, m.p. 142—144°C. Conversion to the free base is done by partitioning the salt between 3N sodium hydroxide and methylene chloride. The combined organic layers are dried over potassium carbonate and the solvent removed *in vacuo*. The resulting oil 2.9 g (0.010 mole) is dissolved in 2-propanol and treated with an equimolar amount of fumaric acid in the same solvent. Recrystallization from 2-propanol/ether gives 4.08 g (99%) of pure *N*-(3-Methyl-2-oxazolidinylidene)-*N'*-phenyl-1-pyrrolidine-carboximidamide fumarate, m.p. 160—162°C.

## Example XL

*N*-(2,3,5,6-Tetrahydro-4-methyl-1,4-oxazine-3-ylidene)-*N'*-phenyl-1-pyrrolidinecarboximidamide fumarate:

Triethyloxonium fluoroborate is prepared from 3.70 g (0.040 mole) of epichlorohydrin in 14 ml of ether and 7.58 g (0.054 mole) of boron trifluoride etherate in 6 ml of ether. The solid triethyloxonium fluoroborate is dissolved in 20 ml of dry methylene chloride and treated under nitrogen with 4.60 g (0.040 mole) of 4-methyl-1,4-oxazine-2-one in 20 ml of dry methylene chloride. The reaction mixture is stirred under nitrogen at room temperature overnight. A 50 ml methylene chloride solution of N-phenyl-1-pyrrolidinecarboximidamide free base (obtained from 12.7 g, 0.040 mole of the corresponding hydroiodide with 50% sodium hydroxide and dried over potassium carbonate) is added to the reaction mixture and stirred at room temperature overnight.

The solvent is removed *in vacuo* and the resulting $HBF_4$ salt is recrystallized from 2-propanol/ether (1:1) to give 10.3 g of white solid. Conversion to the free base is done by partitioning the salt between 3N sodium hydroxide and methylene chloride. The combined organic layers are dried over potassium carbonate and the solvent removed *in vacuo*. The resulting oil, 9.0 g (0.0315 mole, 78%) is dissolved in 2-propanol and treated with an equimolar amount of fumaric acid in the same solvent. The salt is recrystallized from ethanol-ether to give 8.5 g of pure *N*-(2,3,5,6-Tetrahydro-4-methyl-1,4-oxazine-3-ylidene)-*N'*-phenyl-1-pyrrolidinecarboximidamide fumarate, m.p. 191—193°C.

## Example XLI

By following the procedure of Example XXXIX products having the ring function, 3-methyl-2-oxazolidinylidene, and the procedure of Example XL for products having the ring function, 2,3,5,6-tetrahydro-4-methyl-1,4-oxazine-3-ylidene, except that an equivalent amount of an appropriate guanidine of formula (III) is substituted for the N-phenyl-1-pyrrolidinecarboximidamide of each Example, the following respective products of formula (I) are obtained, converted to the indicated acid addition (HX) salt.

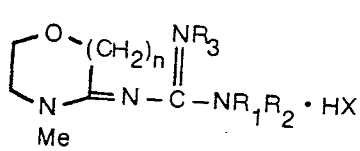

n=zero:

| No. | $R_3$ | $-NR_1R_2$ | HX |
|---|---|---|---|
| 1 | Ph | 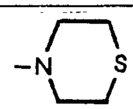 | HI |
| 2 | 3,4-methylenedioxy-Ph | 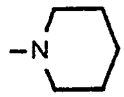 | HBr |
| 3 | 4-*n*-Bu-Ph | $-NEt_2$ | fumarate |
| 4 | 4-OBz-Ph | 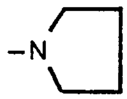 | HI |
| 5 | 4-Me-Ph | 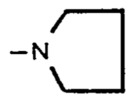 | HCl |
| 6 | 3-Cl-Ph | 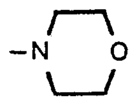 | $H_3PO_4$ |
| 7 | $4-NO_2Ph$ | 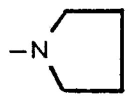 | $H_2SO_4$ |
| 8 | benzhydryl | 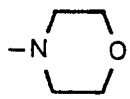 | maleate |
| 9 | *exo*-2-norbornyl | 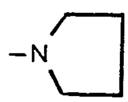 | HI |
| 10 | 3,4-diOMe-Ph | $-NMe_2$ | TsOH |
| 11 | $\alpha,\alpha$-diMe-Bz | $-NEt_2$ | fumarate |
| 12 | 3,4-diCl-Ph | 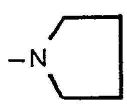 | $H_3PO_4$ |
| 13 | 1-naphthyl | −N (Me) 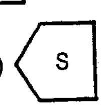 | HI |
| 14 | 4-OMe-Ph | 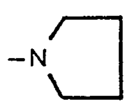 | HI |
| 15 | Bz | 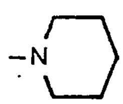 | HI |
| 16 | cyclopentyl | −N (Me) Bz | fumarate |
| 17 | 3,4,5-triOMe-Ph | 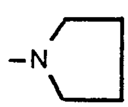 | HI |
| n=1 | | | |
| 18 | Ph | −N (Me) 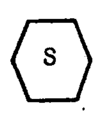 | HI |

| No. | $R_3$ | $—NR_1R_2$ | HX |
|---|---|---|---|
| 19 | *endo*-2-norbornyl | $-NEt_2$ | HBr |
| 20 | $3-CF_3-Ph$ | 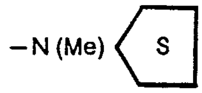 | $HNO_3$ |
| 21 | 4-OMe-Ph | 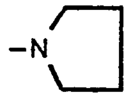 | HI |
| 22 | 4-SMe-Ph | 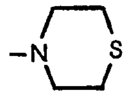 | fumarate |
| 23 | $4-NO_2-Ph$ | 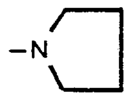 | maleate |
| 24 | 4-OBz-Ph | 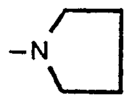 | HI |
| 25 | d,l-$\alpha$-Me-Bz | 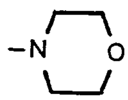 | HI |
| 26 | cyclohexyl | $-NEt_2$ | succinate |
| 27 | *tert*-octyl | 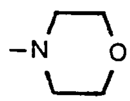 | HCl |
| 28 | 1,2-diphenylethyl | 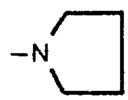 | $HNO_3$ |
| 29 | 4-OMe-2,5-diMe-Ph | $-NMe_2$ | HCl |
| 30 | $\alpha,\alpha$-diMe-phenethyl | 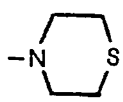 | $HNO_3$ |
| 31 | 1-adamantyl | 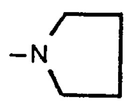 | HI |
| 32 | 3,4-diCl-Ph | $-NEt_2$ | HCl |
| 33 | 1-naphthyl | – N (Me) Bz | fumarate |
| 34 | neopentyl | 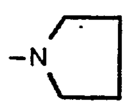 | HI |
| 35 | 2,4,5-triCl-Ph | $-NEt_2$ | fumarate |

Example XLII

*N*-(3-Methyl-2-oxazolidinylidene)-*N'*-phenyl-1-pyrrolidine-carboximidamide fumarate:

To a solution of 3-methyloxazolidin-2-one, 2.02 g (0.02 mole), in dry methylene chloride under dry nitrogen is added 2.28 g (0.02 mole) of methyl fluorosulfonate in one protion. After stirring 3 hrs, 4.51 g (0.02 mole) of N-phenyl-1-pyrrolidinecarboximidamide free base in dry methylene chloride is added. After stirring at room temperature overnight, the solution is shaken with excess cold 3N NaOH.

The organic layer is separated and dried over $K_2CO_3$, filtered, and the solvent removed *in vacuo* to afford the crude base. Addition of an equimolar amount of fumaric acid to a solution of the free base in isopropanol, followed by recrystallization from isopropanol/ether affords pure N-(3-methyl-2-oxazolidinylidene)-N'-phenyl-1-pyrrolidinecarboximidamide fumarate; m.p. 160—162°C.

Example XLIII

N-[(2,3,5,6-Tetrahydro-4-methyl)-1,4-thiazin-3-ylidene]-N'-(4-methoxyphenyl)-1-piperidinecarboximidamide hydroiodide:

Triethyloxonium fluoborate (0.07 mole) is prepared from 13.2 g (0.093 mole) of boron trifluoride etherate and 6.48 g (0.07 mole) of epichlorohydrin in anhydrous ether under dry nitrogen. The resultant oily crystals are washed with fresh dry ether by decantation and dissolved in dry methylene chloride. To this solution is added 4.25 g (0.0324 mole) of 4-methyl-thiamorpholin-3-one (2,3,5,6-tetrahydro-4-methyl-1,4-thiazin-3-one) and the mixture is stirred 2 hr at room temperature. Then 0.027 mole of N-(4-methoxyphenyl)-1-piperidinecarboximidamide in 50 ml of dry $CH_2Cl_2$ and 11.2 g (0.08 mole) of anhydrous potassium carbonate are added. The resulting mixture is stirred overnight at ambient temperatures.

The reaction mixture is filtered and the filtrate is shaken with cold 20% aqueous NaOH. The organic layer is separated and dried over $K_2CO_3$, filtered and the solvent removed *in vacuo* to give an oily residue. Kugelrohr distillation (air bath temperature 120—200°C) removes any unchanged N-(4-methoxyphenyl)-1-piperidinecarboximidamide. The residue of desired product in base form is converted to the HI salt and recrystallized to give pure N-[(2,3,5,6-tetrahydro-4-methyl)-1,4-thiazin-3-yl-idene]-N'-(4-methoxyphenyl)-1-piperidinecarboximidamide hydroiodide.

Example XLIV

The procedure of Example XLIII is followed except that an equivalent quantity of an appropriate guanidine of formula (VII) is substituted for the N-(4-methoxyphenyl)-1-piperidinecarboximidamide used therein to yield the following respective products of formula (I), converted to the indicated acid addition (HX) salt.

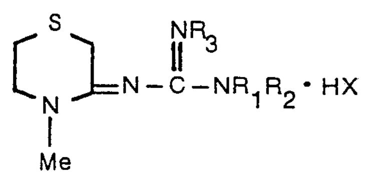

| No. | $R_3$ | —$NR_1R_2$ | HX |
|---|---|---|---|
| 1 | Ph | $-NEt_2$ | HI |
| 2 | benzhydryl | 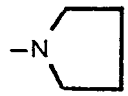 | -base- |
| 3 | 3-Cl-Ph | 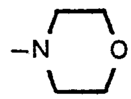 | fumarate |
| 4 | 2,4,5-triCl-Ph | 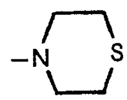 | HCl |
| 5 | Bz | $-NMe_2$ | HCl |
| 6 | 1-naphthyl | 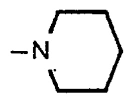 | HBr |
| 7 | do-$\alpha$-Me-Bz | 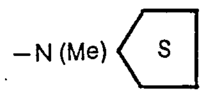 | -base- |
| 8 | $4-NO_2$-Ph | –N (Me) Bz | HI |
| 9 | 3,4-diOMe-Ph | 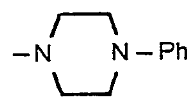 | HCl |
| 10 | cyclohexyl | 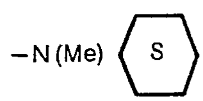 | HCl |

Example XLV

N-[(2,3,5,6-Tetrahydro-4-methyl)-1,4-thiazin-3-ylidene]-N'-hydroxyphenyl)-1-piperidinecarboximidamide hydroiodide:

A solution of 5.03 g (0.01 mole) of the $R_3$=4-methoxyphenyl derivative of Example XLIII in 7 g of 50% HI and 7 g of glacial acetic acid is heated under reflux for 6 hr. The solvent and excess HI are removed *in vacuo* to yield the product, N-[(2,3,5,6-tetrahydro-4-methyl)-1,4-thiazin-3-ylidene]-N'-(4-hydroxyphenyl)-1-piperidinecarboximidamide hydroiodide.

Example XLVI

By following the hydrolysis procedure of Example XLV except that an equivalent amount of an appropriate $R_3$=4-methoxyphenyl derivative is utilized as the precursor to be hydrolyzed, the following respective $R_3$=hydroxyphenyl derivatives of formula (I) are obtained (converted to free base):

1. Precursor: Compound No. 14 of Example XXXVIII
   Product: N-(4-Hydroxyphenyl)-N'-(3-methyl-2-thiazolidinylidene)-1-morpholinecarboximidamide.

2. Precursor: Compound No. 14 of Example XLI
   Product: N-(4-Hydroxyphenyl)-N'-(3-methyl-2-oxazolidinylidene)-1-pyrrolidinecarboximidamide.

3. Precursor: Compound No. 20 of Example XLI
   Product: N-(4-Hydroxyphenyl)-N'-(2,3,5,6-tetrahydro-4-methyl-1,4-oxazine-3-ylidene)-1-pyrrolidinecarboximidamide.

### Example XLVII

N-(4-Hydroxyphenyl)-N'-(3-methyl-2-thiazolidinylidene)-1-piperidinecarboximidamide hydroiodide:

To 2.56 g (7.7 mmoles) of the $R_3$=4-methoxyphenyl derivatives of Example XXXVII (as the free base) is added 4.92 g (19.2 mmoles) of 50% HI and 4.0 g of glacial acetic acid. The resulting mixture is heated (oil bath) under reflux overnight. The excess HI and acetic acid are removed *in vacuo* and the residue is washed several times with ether and then scratched to afford crystals which are suspended in *t*-BuOH, filtered, and washed with ether to furnish the crude product; m.p. 174—176°C. Recrystallization from *t*-BuOH (containing a little MeOH to hep dissolve the crystals and then boiling off the MeOH) gives pure N-(4-hydroxyphenyl)-N'-(3-methyl-2-thiazolidinylidene)-1-piperidinecarboximidamide hydroiodide which is dried *in vacuo*; m.p. 175—177°C.

### Example XLVIII

N-(4-Acetyloxyphenyl)-N'-(3-methyl-2-oxazolidinylidene)-1-pyrrolidinecarboximidamide hydroiodide:

A solution of 4.16 g (0.01 mole) of N-(4-hydroxyphenyl)-N'-(3-methyl-2-oxazolidinylidene)-1-pyrrolidinecarboximideamide hydroiodide in 10 ml of acetic anhydride and 20 ml of methylene chloride is treated with 20.6 g (0.1 mole) of dicyclohexylcarbodiimide under argon. After stirring at ambient temperatures overnight, the formed dicyclohexylurea is removed by filtration and the filtrate is taken to dryness *in vacuo*. Trituration with ether removes excess carbodiimide affording as the residue N-(4-acetyloxyphenyl)-N'-(3-methyl-2-oxazolidinylidene)-1-pyrrolidinecarboximidamide hydroiodide.

### Example IL

The O-acylation procedure of Example XLVIII is followed, except that an equivalent amount of an appropriate $R_3$=4-hydroxyphenyl derivative of Examples XLV and XLVI is used as the starting material to be acylated, to yield as respective products the following $R_3$=acetyloxyphenyl derivatives of formula (I):

1. N-(acetyloxyphenyl)-N'-[(2,3,5,6-tetrahydro)-4-methyl]-1,4-thiazine-3-ylidine-1-piperidinecarboximidamide HI.
2. N-(acetyloxyphenyl)-N'-(3-methyl-2-thiazolidinylidene)-4-morpholinecarboximidamide HI.
3. N-(acetyloxyphenyl)-N'-(2,3,5,6-tetrahydro-4-methyl-1,4-oxazine-3-ylidene)-1-pyrrolidinecarboximidamide HI.

**Claims for the Contracting States: BE CH DE FR GB IT NL SE**

1. A heterocyclic derivative of guanidine selected from compounds having the formula:

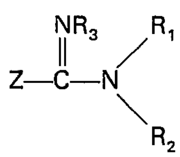

and the pharmaceutically acceptable acid addition salts thereof wherein;

Z is a member selected from

(a) 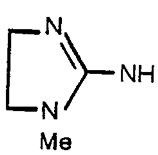 (b) 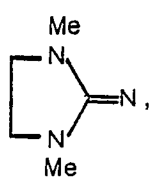, (c) 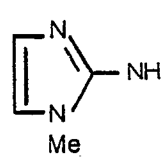

(d) 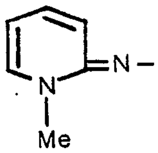 (e) 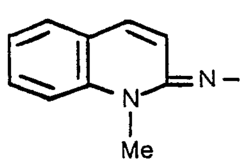 and (f) 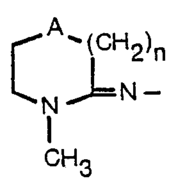

wherein:

A is O or S;

n is zero or 1;

$R_1$ is methyl or ethyl;

$R_2$ is $C_1$—$C_4$ alkyl, cycloalkyl having 3 to 6 carbons or benzyl;

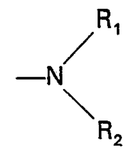

taken together represents

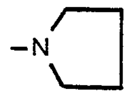 , 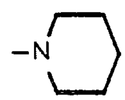 , or 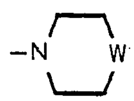

wherein W is O, S, N-$C_1$—$C_4$ alkyl or N-phenyl; and

$R_3$ is alkyl having from 4 to 10 carbons;

phenyl; methylenedioxyphenyl; phenyl substituted with from 1 to 3 substituents each selected from halo, $C_1$—$C_4$ alkyl, and $C_1$—$C_4$ alkoxy; and phenyl substituted with hydroxy, benzyloxy, nitro; trifluoromethyl or methylthio; naphthyl; cycloalkyl having from 5 to 8 carbons; bicycloalkyl having from 7 to 10 carbons; tricycloalkyl having 9 or 10 carbons;

arylalkyl in which the aryl function is phenyl or naphthyl and the alkyl function has from 1 to 4 carbons; or

diphenylalkyl in which the alkyl function has 1 or 2 carbons.

2. A compound selected from N-(1-methyl-2-imidazolidinylidene)-N′-phenyl-1-pyrrolidinecarboxidamide and the pharmaceutically acceptable acid addition salts thereof.

3. A compound selected from N-(1,3-dimethyl-2-imidazolidinylidene)-N′-phenyl-1-pyrrolidinecarboximidamide and the pharmaceutically acceptable acid addition salts thereof.

4. A compound selected from N-(1-methylimidazol-2-yl)-N′-phenyl-4-morpholine-carboximidamide and the pharmaceutically acceptable acid addition salts thereof.

5. A compound selected from N-(1-adamantyl)-N′-(1-methylimidazol-2-yl)-1-pyrrolidinecarboximidamide and the pharmaceutically acceptable acid addition salts thereof.

6. A compound selected from N-(1,2-dihydro-1-methyl-2(1*H*)pyridinylidene)-N′-phenyl-1-pyrrolidinecarboximidamide and the pharmaceutically acceptable acid addition salts thereof.

7. A compound selected from N-(1,2-dihydro-1-methyl-2(1*H*)quinolinylidene)-N′-phenyl-1-pyrrolidinecarboximidamide and the pharmaceutically acceptable acid addition salts thereof.

8. A compound selected from N-(3-methyl-2-thiazolidinylidene)-N′-phenyl-1-pyrrolidinecarboximidamide and the pharmaceutically acceptable acid addition salts thereof.

9. A compound selected from N-(3-methyl-2-oxazolidinylidene)-N′-phenyl-1-pyrrolidinecarboximidamide and the pharmaceutically acceptable acid addition salts thereof.

10. A compound selected from N-(2,3,5,6-tetrahydro-4-methyl-1,4-oxazine-3-ylidene)-N′-phenyl-1-pyrrolidinecarboximidamide and the pharmaceutically acceptable acid addition salts thereof.

11. A compound selected from N-[(2,3,5,6-tetrahydro-4-methyl)-1,4-thiazin-3-ylidene]-N′-(4-methoxyphenyl)-1-piperidinecarboximidamide and the pharmaceutically acceptable acid addition salts thereof.

12. A process for preparing a heterocyclic derivative of guanidine as claimed in claim 1 characterized by (a) reacting a compound of the formula

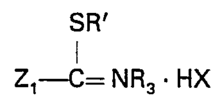

(V)

wherein $Z_1$ is the same as Z, but other than (d) or (e), and in the case of (f) A is sulfur and n is zero; and wherein $R_3$ is as previously described other than hydroxyphenyl, and in the instance wherein $Z_1$ is (f), $R_3$ is as previously described, but other than hydroxyphenyl and $C_1$—$C_4$ alkanoyloxyphenyl; and wherein HX is an acid addition salt, with a compound of the formula $HNR_1R_2$, wherein $R_1$, $R_2$ and $NR_1R_2$ are as previously described, to yield the guanidine derivatives of formula (I), in similar acid addition form, which are readily obtained as the corresponding base form by conventional treatment with suitable alkali; or (b) preparing formula (I) compounds wherein $Z_1$ is (f) and wherein $R_3$ is hydroxyphenyl by hydrolysis of the corresponding $R_3$=methoxyphenyl derivatives, and acylation of the resultant $R_3$=hydroxyphenyl derivatives by glacial acetic acid in the presence of excess dicyclohexylcarbodiimide to afford the corresponding $R_3$=$C_1$—$C_4$ alkanoyloxy derivatives of formula (I) [and in which $Z_1$ is (f)]; or (c) preparing compounds of formula (I), wherein $R_1$, $R_2$, $NR_1R_2$ and $R_3$ (other than hydroxyphenyl) are as previously defined and Z is equal to the functions represented by letters (b), (d), (e), and (f), by reacting

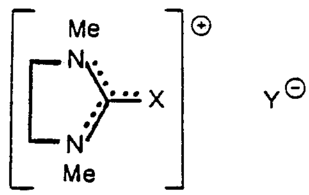

(VI)

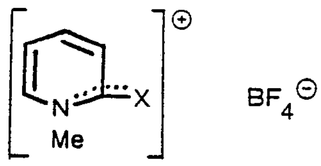

(XV)

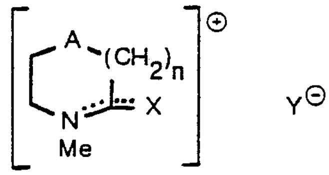

(IXX)

wherein X is either methoxy or ethoxy and $Y^{\theta}$ is either $BF_4^{\theta}$ or $OSO_2F^{\theta}$, with a compound of the formula

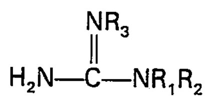

(VII)

in order to prepare a compound of the formula

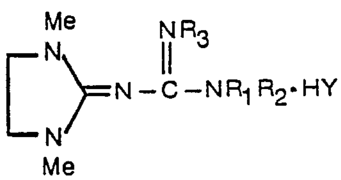

(VIII)

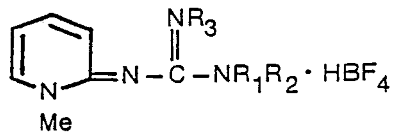

(XVI)

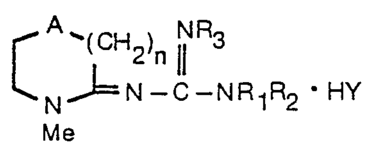

(XX)

respectively, and, if desired, the products (VIII), (XVI) or (XX), in the form of the corresponding salt, is converted to the corresponding base form (I); or (d) preparing the formula (I) compounds wherein Z is (a), (b) or (c), Z in such case being designated $Z_2$, characterized by reacting a compound of the formula

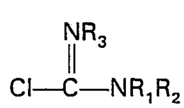

with a compound of the formula $Z_2H$, and if desired, the formula (I) compounds wherein $R_3$ is hydroxyphenyl may be prepared either (i) by debenzylation of the corresponding $R_3$=benzyloxyphenyl derivatives or (ii) by hydrolysis of the corresponding $R_3$=benzyloxyphenyl and methoxyphenyl derivatives.

13. A pharmaceutical formulation comprising a compound according to any one of Claims 1 to 11.

14. A compound according to any one of Claims 1 to 11, or a formulation according to Claim 13, for use as a hypoglycemic agent.

**Claims for the Contracting State: AT**

1. A process for preparing a heterocyclic derivative of guanidine selected from compounds having the formula:

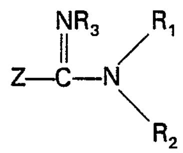

and the pharmaceutically acceptable acid addition salts thereof wherein:

Z is a member selected from

(a) 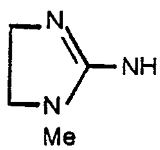 (b) 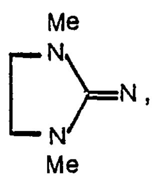 , (c) 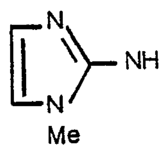

(d) 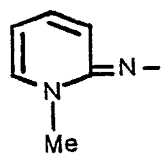 (e) 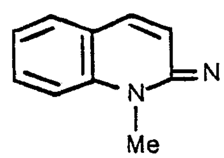 and (f) 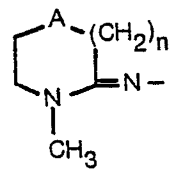

wherein:

A is O or S;

n is zero or 1;

$R_1$ is methyl or ethyl;

$R_2$ is $C_1$—$C_4$ alkyl, cycloalkyl having from 3 to 6 carbons; or benzyl;

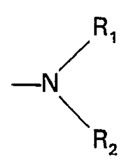

taken together, represents

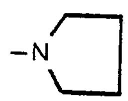 , 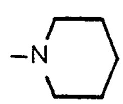 , or 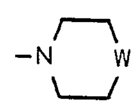

wherein W is O, S, N-$C_1$—$C_4$ alkyl or N-phenyl; and

$R_3$ is alkyl having from 4 to 10 carbons;

phenyl; methylenedioxyphenyl; phenyl substituted with from 1 to 3 substituents each selected from halo, $C_1$—$C_4$ alkyl, and $C_1$—$C_4$ alkoxy; and phenyl substituted with hydroxy, benzyloxy,. nitro; trifluoromethyl or methylthio; naphthyl; cycloalkyl having from 5 to 8 carbons; bicycloalkyl having from 7 to 10 carbons; tricycloalkyl having 9 or 10 carbons; arylalkyl in which the aryl function is selected from phenyl and naphthyl and the alkyl function has from 1 to 4 carbons; or

diphenylalkyl in which the alkyl function has 1 or 2 carbons, characterized by (a) reacting a compound of the formula

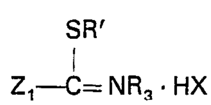 (V)

wherein $Z_1$ is the same as Z, but other than (d) or (e), and in the case of (f) A is sulfur and n is zero; and wherein $R_3$ is as previously described other than hydroxyphenyl, and in the instance wherein $Z_1$ is (f), $R_3$ is as previously described, but other than hydroxyphenyl and $C_1$—$C_4$-alkanoyloxyphenyl; and wherein HX is an acid addition salt, with a compound of the formula $NHR_1R_2$, wherein $R_1$, $R_2$ and $NR_1R_2$ are as previously described, to yield the guanidine derivatives of formula (I), in similar acid addition form, which are readily obtained as the corresponding base form by conventional treatment with suitable alkali; or (b) preparing formula (I) compounds wherein $Z_1$ is (f) and wherein $R_3$ is hydroxyphenyl by hydrolysis of the corresponding $R_3$=methoxyphenyl derivatives and acylation of the resultant $R_3$=hydroxyphenyl derivatives by glacial acetic acid in the presence of excess dicyclohexylcarbodiimide to afford the corresponding $R_3$=$C_1$—$C_4$ alkanoyloxy derivatives of formula (I) [and in which $Z_1$ is (f)]; or (c) preparing compounds of formula (I), wherein R, $R_2$, $NR_1R_2$ and $R_3$ (other than hydroxyphenyl) are as previously defined and Z is equal to the functions represented by letters (b), (d), (e), and (f), by reacting

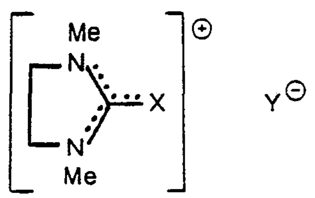

(VI)

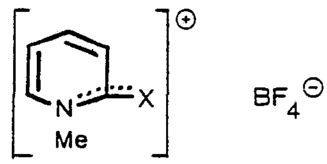

(XV)

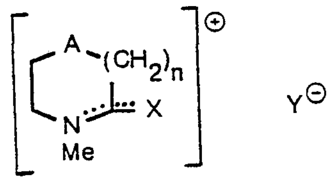

(IXX)

wherein X is either methoxy or ethoxy and $Y^\theta$ is either $BF_4^\theta$ or $OSO_2F^\theta$, with a compound of the formula

$$H_2N—\overset{\overset{\displaystyle NR_3}{\|}}{C}—NR_1R_2 \qquad (VII)$$

in order to prepare a compound of the formula

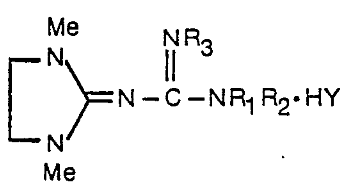

(VIII)

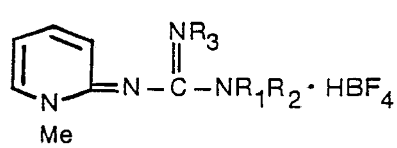

(XVI)

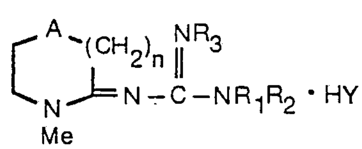

(XX)

respectively, and, if desired, the products (VIII), (XVI) or (XX), in the form of the corresponding salt, is converted to the corresponding base form (I); or (d) preparing the formula (I) compounds wherein Z is (a), (b) or (c), Z in such case being designated $Z_2$, characterized by reacting a compound of the formula

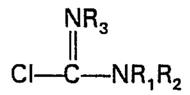

with a compound of the formula $Z_2H$, and if desired, the formula (I) compounds wherein $R_3$ is hydroxyphenyl may be prepared either (i) by debenzylation of the corresponding $R_3$=benzyloxyphenyl derivatives or (ii) by hydrolysis of the corresponding $R_3$=benzyloxyphenyl and methoxyphenyl derivatives.

2. A process according to Claim 1 for preparing a compound selected from N-(1-methyl-2-imidazolidinylidene)-N'-phenyl-1-pyrrolidinecarboximidamide and the pharmaceutically acceptable acid addition salts thereof.

3. A process according to Claim 1 for preparing a compound selected from N-(1,3-dimethyl-2-imidazolidinylidene)-N'-phenyl-1-pyrrolidinecarboximidamide and the pharmaceutically acceptable acid addition salts thereof.

4. A process according to Claim 1 for preparing a compound selected from N-(1-methylimidazol-2-yl)-N'-phenyl-4-morpholinecarboximidamide and the pharmaceutically acceptable acid addition salts thereof.

5. A process according to Claim 1 for preparing a compound selected from N-(1-adamantyl)-N'-(1-methylimidazol-2-yl)-1-pyrrolidinecarboximidamide and the pharmaceutically acceptable acid addition salts thereof.

6. A process according to Claim 1 for preparing a compound selected from N-(1,2-dihydro-1-methyl-2(1*H*)pyridinylidene)-N'-phenyl-1-pyrrolidinecarboximidamide and the pharmaceutically acceptable acid addition salts thereof.

7. A process according to Claim 1 for preparing a compound selected from N-(1,2-dihydro-1-methyl-2(1*H*)quinolinylidene)-N'-phenyl-1-pyrrolidinecarboximidamide and the pharmaceutically acceptable acid addition salts thereof.

8. A process according to Claim 1 for preparing a compound selected from N-(3-methyl-2-thiazolidinylidene)-N'-phenyl-1-pyrrolidinecarboximidamide and the pharmaceutically acceptable acid addition salts thereof.

9. A process according to Claim 1 for preparing a compound selected from N-(3-methyl-2-oxazolidinylidene)-N'-phenyl-1-pyrrolidinecarboximidamide and the pharmaceutically acceptable acid addition salts thereof.

10. A process according to Claim 1 for preparing a compound selected from N-(2,3,5,6-tetrahydro-4-methyl-1,4-oxazine-3-ylidene)-N'-phenyl-1-pyrrolidinecarboximidamide and the pharmaceutically acceptable acid addition salts thereof.

11. A process according to Claim 1 for preparing a compound selected from N-[(2,3,5,6-tetrahydro-4-methyl)-1,4-thiazin-3-ylidene]-N'-(4-methoxyphenyl)-1-piperidinecarboximidamide and the pharmaceutically acceptable acid addition salts thereof.

12. A process for preparing a pharmaceutical composition, characterized by formulating a compound obtained by a process according to any of Claims 1 to 11 into conventional liquid and solid pharmaceutical dosage forms and preparations.

**Patentansprüche für die Vertragsstaaten: BE CH DE FR GB IT NL SE**

1. Ein heterocyclisches Derivat von Guanidin, ausgewählt unter Verbindungen mit der Formel:

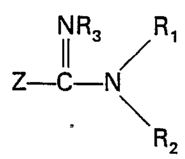

und den pharmazeutisch annehmbaren Säureadditionssalzen hievon, worin

Z für einen unter

(a) 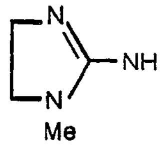 (b) 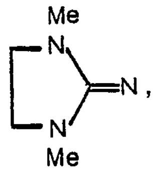, (c) 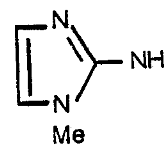

(d) 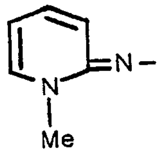 (e) 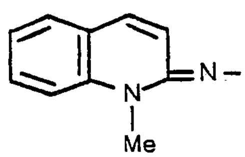 und (f) 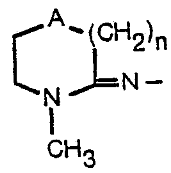

ausgewählten Vertreter steht, worin:

A Sauerstoff oder Schwefel bedeutet;

n Null oder 1 bedeutet;

$R_1$ Methyl oder Ethyl darstellt;

$R_2$ für $C_1$—$C_4$-Alkyl, Cycloalkyl mit 3 bis 36 Kohlenstoffatomen oder Benzyl steht;

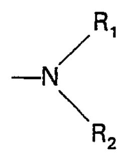

zusammen für

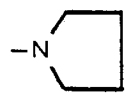, 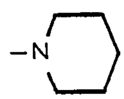, or 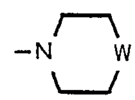

steht, worin W für Sauerstoff, Schwefel, N-$C_1$—$C_4$-Alkyl oder N-Phenyl steht; und

$R_3$ für

Alkyl mit 4 bis 10 Kohlenstoffatomen; Phenyl; Methylendioxyphenyl; mit 1 bis 3 Substituenten, jeweils ausgewählt unter Halogen, $C_1$—$C_4$-Alkyl und $C_1$—$C_4$-Alkoxy substituiertes Phenyl; und mit Hydroxy, Benzyloxy, Nitro; Trifluormethyl oder Methylthio substituiertes Phenyl; Naphthyl; Cycloalkyl mit 5 bis 8 Kohlenstoffatomen; Bicycloalkyl mit 7 bis 10 Kohlenstoffatomen; Tricycloalkyl mit 9 oder 10 Kohlenstoffatomen; Arylalkyl, worin die Arylfunktion Phenyl oder Naphthyl ist und die Alkylfunktion 1 bis 4 Kohlenstoffatome aufweist; oder

Diphenylalkyl steht, worin die Alkylfunktion 1 oder 2 Kohlenstoffatome aufweist.

2. Eine Verbindung, ausgewählt unter N-(1-Methyl-2-imidazolidinyliden)-N′-phenyl-1-pyrrolidincarboximidamid und den pharmazeutisch annehmbaren Säureadditionssalzen hievon.

3. Eine Verbindung, ausgewählt unter N-(1,3-Dimethyl-2-imidazolidinyliden)-N′-phenyl-1-pyrrolidincarboximidamid und den pharmazeutisch annehmbaren Säureadditionssalzen hievon.

4. Eine Verbindung, ausgewählt unter N-(1-Methylimidazol-2-yl)-N′-phenyl-4-morpholincarboximidamid und den pharmazeutisch annehmbaren Säureadditionssalzen hievon.

5. Eine Verbindung, ausgewählt unter N-(1-Adamantyl)-N′-(1-methylimidazol-2-yl)-1-pyrrolidincarboximidamid und den pharmazeutisch annehmbaren Säureadditionssalzen hievon.

6. Eine Verbindung, ausgewählt unter N-(1,2-Dihydro-1-methyl-2-(1*H*)pyridinyliden)-N′-phenyl-1-pyrrolidincarboximidamid und den pharmazeutisch annehmbaren Säureadditionssalzen hievon.

7. Eine Verbindung, ausgewählt unter N-(1,2-Dihydro-1-methyl-2-(1*H*)chinolinyliden)-N′-phenyl-1-pyrrolidincarboximidamid und den pharmazeutisch annehmbaren Säureadditionssalzen hievon.

8. Eine Verbindung, ausgewählt unter N-(3-Methyl-2-thiazolidinyliden)-N′-phenyl-1-pyrrolidincarboximidamid und den pharmazeutisch annehmbaren Säureadditionssalzen hievon.

9. Eine Verbindung, ausgewählt unter N-(3-Methyl-2-oxazolidinyliden)-N′-phenyl-1-pyrrolidincarboximidamid und den pharmazeutisch annehmbaren Säureadditionssalzen hievon.

10. Eine Verbindung, ausgewählt unter N-(2,3,5,6-Tetrahydro-4-methyl-1,4-oxazin-3-yliden)-N′-phenyl-1-pyrrolidincarboximidamid und den pharmazeutisch annehmbaren Säureadditionssalzen hievon.

11. Eine Verbindung, ausgewählt unter N-[(2,3,5,6-Tetrahydro-4-methyl)-1,4-thiazin-3-yliden]-N′-4-methoxyphenyl)-1-piperidincarboximidamid und den pharmazeutisch annehmbaren Säureadditionssalzen hievon.

12. Ein Verfahren zur Herstellung eines heterocyclischen Guanidinderivats wie in Anspruch 1 beansprucht, gekennzeichnet durch (a) Umsetzen einer Verbindung der Formel

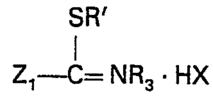

(V)

worin $Z_1$ die gleiche Bedeutung wie Z hat, jedoch eine andere Bedeutung als (d) oder (e) aufweist, und im Falle (f) A Schwefel bedeutet und n Null ist; und worin $R_3$ wie zuvor beschrieben ist, jedoch eine andere Bedeutung als Hydroxyphenyl aufweist, und im Falle daß $Z_1$ die Bedeutung (f) aufweist, $R_3$ wie zuvor beschrieben ist, jedoch eine andere Bedeutung als Hydroxyphenyl und $C_1$—$C_4$-Alkanoyloxyphenyl hat; und worin HX ein Säureadditionssalz bedeutet, mit einer Verbindung der Formel $HNR_1R_2$, worin $R_1$, $R_2$ und $NR_1R_2$ wie zuvor beschrieben sind, zur Ausbildung der Guanidinderivate der Formel (I), in ähnlicher Säureadditionsform, welche leicht als die entsprechende Basenform durch konventionelle Behandlung mit geeignetem Alkali erhalten werden; oder (b) Bereiten von Verbindungen der Formel (I), worin $Z_1$ die Bedeutung (f) hat und worin $R_3$ Hydroxyphenyl ist, durch Hydrolyse der entsprechenden $R_3$=Methoxyphenyl-Derivate und Acylieren der gebildeten $R_3$=Hydroxyphenyl-Derivate mit Eisessig in Gegenwart von überschüssigem Dicyclohexylcarbodiimid unter Ausbildung der entsprechenden $R_3$=$C_1$—$C_4$-Alkanoyloxy-Derivate der Formel (I) [worin $Z_1$ die Bedeutung (f) aufweist]; oder (c) Bereiten von Verbindungen der Formel (I), worin $R_1$, $R_2$, $NR_1R_2$ und $R_3$ (mit anderer Bedeutung als Hydroxyphenyl) wie zuvor definiert sind und Z den durch die Buchstaben (b), (d), (e) und (f) dargestellten Funktionen entspricht, durch Umsetzen von

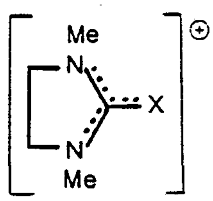

$Y^{\ominus}$

(VI)

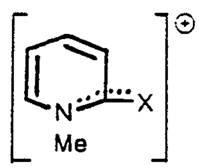

$BF_4^{\ominus}$

(XV)

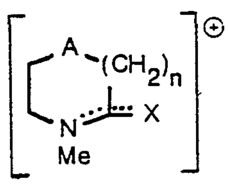

$Y^{\ominus}$

(IXX)

worin X entweder Methoxy oder Ethoxy bedeutet und $Y^{\ominus}$ entweder $BF_4^{\ominus}$ oder $OSO_2F^{\ominus}$ darstellt, mit einer Verbindung der Formel

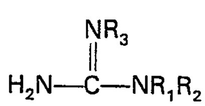

(VII)

zur Ausbildung einer Verbindung der Formel

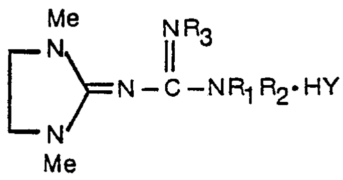

(VIII)

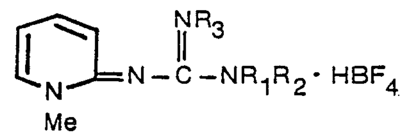

(XVI)

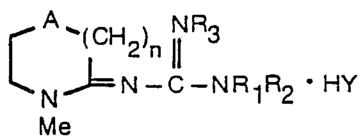

(XX)

und, gewünschtenfalls, Überführen der Produkte (VIII), (XVI) oder (XX), in Form des entsprechenden Salzes, in die entsprechende Basenform (I); oder (d) Bereiten der Verbindungen der Formel (I), worin Z die Bedeutung (a), (b) oder (c) aufweist, in welchem Falle Z durch $Z_2$ bezeichnet wird, durch Umsetzen einer Verbindung der Formel

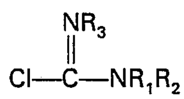

mit einer Verbindung der Formel $Z_2H$ und, gewünschtenfalls, Herstellen von Verbindungen der Formel (I), worin $R_3$ Hydroxyphenyl bedeutet, entweder durch (i) Debenzylierung der korrespondierenden $R_3$=Benzyloxyphenyl-Derivate oder (ii) durch Hydrolyse der korrespondierenden $R_3$=Benzyloxyphenyl- und Methoxyphenyl-Derivate.

13. Eine pharmazeutische Formulierung, umfassend eine Verbindung gemäß einem der Ansprüche 1 bis 11.

14. Eine Verbindung gemäß einem der Ansprüche 1 bis 11, oder eine Formulierung gemäß Anspruch 13, zur Anwendung als ein hypoglykämisches Mittel.

**Patentansprüche für den Vertragsstaat: AT**

1. Ein Verfahren zur Herstellung eines heterocyclischen Derivats von Guanidin, ausgewählt unter Verbindungen mit der Formel:

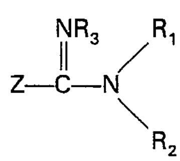

und den pharmazeutisch annehmbaren Säureadditionssalzen hievon, worin

Z für einen unter

(a) 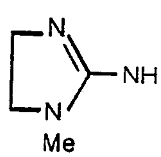 (b) 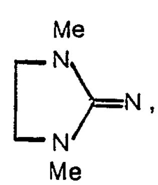, (c) 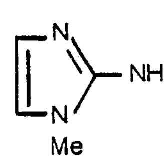

(d) 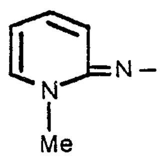 (e) 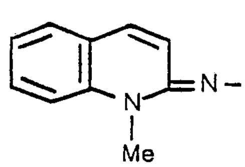 und (f) 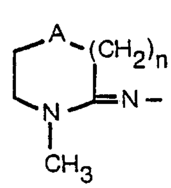

ausgewählten Vertreter steht, worin:

A Sauerstoff oder Schwefel bedeutet;

n Null oder 1 bedeutet;

$R_1$ Methyl oder Ethyl darstellt;

$R_2$ für $C_1$—$C_4$-Alkyl, Cycloalkyl mit 3 bis 6 Kohlenstoffatomen oder Benzyl steht;

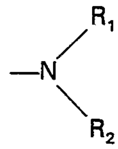

zusammen für

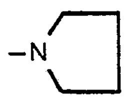 , 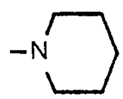 , oder 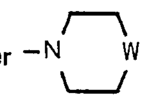

steht, worin W für Sauerstoff, Schwefel, N-$C_1$—$C_4$-Alkyl oder N-Phenyl steht; und

$R_3$ für

Alkyl mit 4 bis 10 Kohlenstoffatomem; Phenyl; Methylendioxyphenyl; mit 1 bis 3 Substitueneten, jeweils ausgewählt unter Halogen, $C_1$—$C_4$-Alkyl und $C_1$—$C_4$-Alkoxy substituiertes Phenyl; und mit Hydroxy, Benzyloxy, Nitro; Trifluormethyl oder Methylthio substituiertes Phenyl; Naphthyl; Cycloalkyl mit 5 bis 8 Kohlenstoffatomen; Bicycloalkyl mit 7 bis 10 Kohlenstoffatomen; Tricycloalkyl mit 9 oder 10 Kohlenstoffatomen; Arylalkyl, worin die Arylfunktion Phenyl oder Naphthyl ist und die Alkylfunktion 1 bis 4 Kohlenstoffatome aufweist; oder

Diphenylalkyl steht, worin die Alkylfunktion 1 oder 2 Kohlenstoffatome aufweist, gekennzeichnet durch

(a) Umsetzen einer Verbindung der Formel

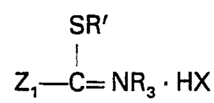 (V)

worin $Z_1$ die gleiche Bedeutung wie Z hat, jedoch eine andere Bedeutung als (d) oder (e) aufweist, und im Falle (f) A Schwefel bedeutet und n Null ist; und worin $R_3$ wie zuvor beschrieben ist, jedoch eine andere Bedeutung als Hydroxyphenyl aufweist, und im Falle daß $Z_1$ die Bedeutung (f) aufweist, $R_3$ wie zuvor beschrieben ist, jedoch eine andere Bedeutung als Hydroxyphenyl und $C_1$—$C_4$-Alkanoyloxyphenyl hat; und worin HX ein Säureadditionssalz bedeutet, mit einer Verbindung der Formel $HNR_1R_2$, worin $R_1$, $R_2$ und $NR_1R_2$ wie zuvor beschrieben sind, zur Ausbildung der Guanidinderivate der Formel (I), in ähnlicher Säureadditionsform, welche leicht als die entsprechende Basenform durch konventionelle Behandlung mit geeignetem Alkali erhalten werden; oder (b) Bereiten von Verbindungen der Formel (I), worin $Z_1$ die Bedeutung (f) hat und worin $R_3$ Hydroxyphenyl ist, durch Hydrolyse der entsprechenden $R_3$=Methoxyphenyl-Derivate und Acylieren der gebildeten $R_3$=Hydroxyphenyl-Derivate mit Eisessig in Gegenwart von überschüssigem Dicyclohexylcarbodiimid under Ausbildung der entsprechenden $R_3$= $C_1$—$C_4$-Alkanoyloxy-Derivate der Formel (I) [worin $Z_1$ die Bedeutung (f) aufweist]; oder (c) Bereiten von Verbindungen der Formel (I), worin $R_1$, $R_2$, $NR_1R_2$ und $R_3$ (mit anderer Bedeutung als Hydroxyphenyl) wie zuvor definiert sind und Z den durch die Buchstaben (b), (d), (e) und (f) dargestellten Funktionen entspricht, durch Umsetzen von

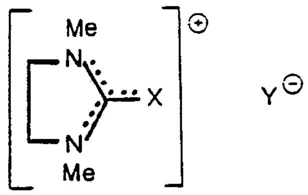

(VI)

oder

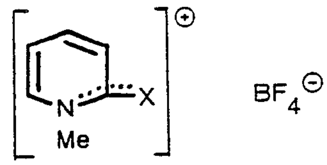

(XV)

oder

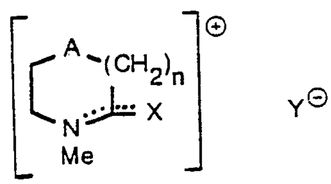

(IXX)

worin X entweder Methoxy oder Ethoxy bedeutet und $Y^{\ominus}$ entweder $BF_4^{\ominus}$ oder $OSO_2F^{\ominus}$ darstellt, mit einer Verbindung der Formel

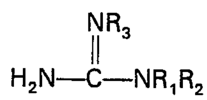

(VII)

zur Ausbildung einer Verbindung der Formel

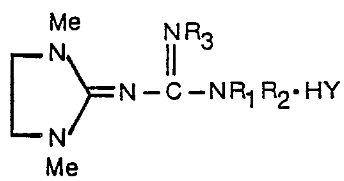

(VIII)

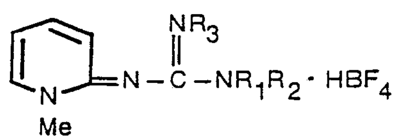

(XVI)

bzw.

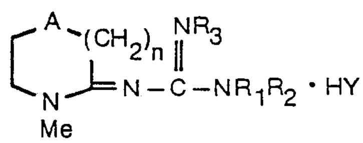

(XX)

und, gewünschtenfalls, Überführen der Produkte (VIII), (XVI) oder (XX), in Form des entsprechenden Salzes, in die entsprechende Basenform (I); oder (d) Bereiten der Verbindungen der Formel (I), worin Z die Bedeutung (a), (b) oder (c) aufweist, in welchem Falle Z durch $Z_2$ bezeichnet wird, durch Umsetzen einer Verbindung der Formel

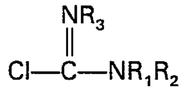

mit einer Verbindung der Formel $Z_2H$ und, gewünschtenfalls, Herstellen von Verbindungen der Formel (I), worin $R_3$ Hydroxyphenyl bedeutet, entweder durch (i) Bebenzylierung der korrespondierenden $R_3$=Benzyloxyphenyl-Derivate oder (ii) durch Hydrolyse der korrespondierenden $R_3$=Benzyloxyphenyl- und Methoxyphenyl-Derivate.

2. Ein Verfahren gemäß Anspruch 1 zur Herstellung einer Verbindung, ausgewählt unter N-(1-Methyl-2-imidazolidinyliden)-N'-phenyl-1-pyrrolidincarboximidamid und den pharmazeutisch annehmbaren Säureadditionssalzen hievon.

3. Ein Verfahren gemäß Anspruch 1 zur Herstellung einer Verbindung, ausgewählt unter N-(1,3-Dimethyl-2-imidazolidinyliden)-N'-phenyl-1-pyrrolidincarboximidamid und den pharmazeutisch annehmbaren Säureadditionssalzen hievon.

4. Ein Verfahren gemäß Anspruch 1 zur Herstellung einer Verbindung, ausgewählt unter N-(1-Methylimidazol-2-yl)-N'-phenyl-4-morpholincarboximidamid und den pharmazeutisch annehmbaren Säureadditionssalzen hievon.

5. Ein Verfahren gemäß Anspruch 1 zur Herstellung einer Verbindung, ausgewählt unter N-(1-Adamantyl)-N'-(1-methyl-imidazol-2-yl)-1-pyrrolidincarboximidamid und den pharmazeutisch annehmbaren Säureadditionssalzen hievon.

6. Ein Verfahren gemäß Anspruch 1 zur Herstellung einer Verbindung, ausgewählt unter N-(1,2-Dihydro-1-methyl-2-(1*H*)pyridinyliden)-N'-phenyl-1-pyrrolidincarboximidamid und den pharmazeutisch annehmbaren Säureadditionssalzen hievon.

7. Ein Verfahren gemäß Anspruch 1 zur Herstellung einer Verbindung, ausgewählt unter N-(1,2-Dihydro-1-methyl-2-(1*H*)chinolinyliden)-N'-phenyl-1-pyrrolidincarboximidamid und den pharmazeutisch annehmbaren Säureadditionssalzen hievon.

8. Ein Verfahren gemäß Anspruch 1 zur Herstellung einer Verbindung, ausgewählt unter N-(3-Methyl-2-thiazolidinyliden)-N'-phenyl-1-pyrrolidincarboximidamid und den pharmazeutisch annehmbaren Säureadditionssalzen hievon.

9. Ein Verfahren gemäß Anspruch 1 zur Herstellung einer Verbindung, ausgewählt unter N-(3-Methyl-2-oxazolidinyliden)-N'-phenyl-1-pyrrolidincarboximidamid und den pharmazeutisch annehmbaren Säureadditionssalzen hievon.

10. Ein Verfahren gemäß Anspruch 1 zur Herstellung einer Verbindung, ausgewählt unter N-(2,3,5,6-Tetrahydro-4-methyl-1,4-oxazin-3-yliden)-N'-phenyl-1-pyrrolidincarboximidamid und den pharmazeutisch annehmbaren Säureadditionssalzen hievon.

11. Ein Verfahren gemäß Anspruch 1 zur Herstellung einer Verbindung, ausgewählt unter N-[(2,3,5,6-Tetrahydro-4-methyl)-1,4-thiazin-3-yliden]-N'-(4-methoxyphenyl)-1-piperidincarboximidamid und den pharmazeutisch annehmbaren Säureadditionssalzen hievon.

12. Ein Verfahren zur Herstellung einer pharmazeutischen Zusammensetzung, gekennzeichnet durch Formulieren einer nach einem Verfahren gemäß einem der Ansprüche 1 bis 11 erhaltenen Verbindung zu konventionellen, flüssigen und festen pharzeutischen Dosisformen und Präparaten.

**Revendications pour les Etats contractants: BE CH DE FR GB IT NL SE**

1. Dérivé hétérocyclique de guanidine choisi parmi les composés ayant pour formule:

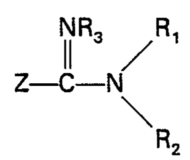

et leurs sels d'addition d'acides acceptables en pharmacie où:

Z est un composant choisi parmi:

(a) 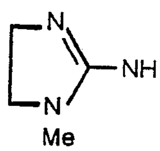 (b) 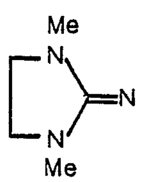, (c) 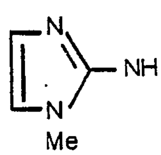

(d) 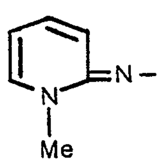 (e) 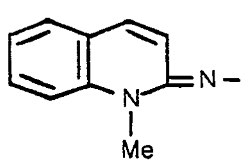 et (f) 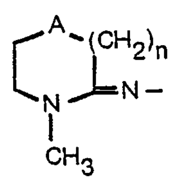

ou:

A est O ou S;

n est zéro ou 1;

$R_1$ est un méthyle ou un éthyle;

$R_2$ est un alkyle en $C_1$—$C_4$, un cycloalkyle ayant 3 à 6 atomes de carbone ou un benzyle;

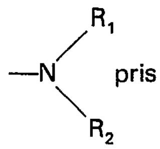 pris ensemble représente

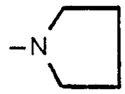 , 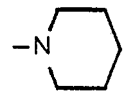 , ou 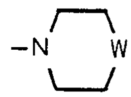

où:

W est O, S, un N-alkyle en $C_1$—$C_4$ ou un N-phényle; et

$R_3$ est un alkyle ayant 4 à 10 atomes de carbone; un phényle; un méthylènedioxyphényle; un phényle substitué par 1 à 3 substituants choisis chacun parmi un halogéno, un alkyle en $C_1$—$C_4$, et un alcoxy en $C_1$—$C_4$; et un phényle substitué par un hydroxy, un benzyloxy; un nitro; un trifluorométhyle ou un méthylthio; un naphtyle un cycloalkyle ayant 5 à 8 atomes de carbone; un bicycloalkyle ayant 7 à 10 atomes de carbone; un tricycloalkyle ayant 9 ou 10 atomes de carbone; un arylalkyle dont la fonction aryle est un phényle ou un naphtyle et la fonction alkyle a 1 à 4 atomes de carbone; ou un diphénylalkyle dont la fonction alkyle a 1 ou 2 atomes de carbone.

2. Composé choisi parmi le N-(1-méthyl-2-imidazolidinylidène)-N'-phényl-1-pyrrolidinecarboxyimidamide et ses sels d'addition d'acides acceptables en pharmacie.

3. Composé choisi parmi le N-(1,3-diméthyl-2-imidazolidinylidène)-N'-phényl-1-pyrrolidinecarboximidamide et ses sels d'addition d'acides acceptables en pharmacie.

4. Composé choisi parmi le N-(1-méthylimidazol-2-yl)-N'-phényl-4-morpholinecarboximidamide et ses sels d'addition d'acides acceptables en pharmacie.

5. Composé choisi parmi le N-(1-adamantyl)-N'-(1-méthylimidazol-2-yl)-1-pyrrolidinecarboximidamide et ses sels d'addition d'acides acceptables en pharmacie.

6. Composé choisi parmi le N-(1,2-dihydro-1-méthyl-2(1*H*)pyridinylidène)-N'-phényl-1-pyrrolidinecarboximidamide et ses sels d'addition d'acides acceptables en pharmacie.

7. Composé choisi parmi le N-(1,2-hydro-1-méthyl-2(1*H*)quinoléinylidène)-N'-phényl-1-pyrrolidinecarboximidamide et ses sels d'addition d'acides acceptables en pharmacie.

8. Composé choisi parmi le N-(3-méthyl-2-thiazolidinylidène)-N'-phényl-1-pyrrolidinecarboximidamide et ses sels d'addition d'acides acceptables en pharmacie.

9. Composé choisi parmi le N-(3-méthyl-2-oxazolidinylidène)-N'-phényl-1-pyrrolidinecarboximidamide et ses sels d'addition d'acides acceptables en pharmacie.

10. Composé choisi parmi le N-(2,3,5,6-tétrahydro-4-méthyl-1,4-oxazine-3-ylidène)-N'-phényl-1-pyrrolidinecarboximidamide et ses sels d'addition d'acides acceptables en pharmacie.

11. Composé choisi parmi le N-[(2,3,5,6-tétrahydro-4-méthyl-1,4-thiazin-3-ylidène]-N'-(4-méthoxyphényl)-1-pipiéridinecarboximidamide et ses sels d'addition d'acides acceptables en pharmacie.

12. Procédé pour préparer un dérivé hétérocyclique de guanidine comme revendiqué dans la revendication 1, caractérisé par:

(a) la réaction d'un composé de formule:

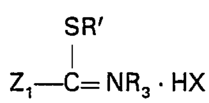 (V)

où $Z_1$ est comme Z mais est autre que (d) ou (e), et dans le case de (f), A est le soufre et n est zéro; et où $R_3$ est comme précédemment décrit, à l'exception d'un hydrophényle, et dans le cas où $Z_1$ est (f), $R_3$ est comme précédemment décrit, à l'exception d'un hydroxyphényle et d'un alcanoyloxy (en $C_1$—$C_4$) phényle; et

où HX est un sel d'addition d'acide, avec un composé de formule $HNR_1R_2$, où $R_1$, $R_2$ et $NR_1R_2$ sont comme précédemment décrits, pour obtenir les dérivés de guanidine de formule (I) sous forme d'un sel d'addition d'acide, qu'on obtient facilement sous forme de la base correspondante par traitement classique avec un alcali approprié; ou

(b) la préparation de composés de formule (I) où $Z_1$ est (f) et où $R_3$ est un hydroxyphényle, par hydrolyse des dérivés correspondants où $R_3$ est un méthoxyphényle, et acylation des dérivés obtenus où $R_3$ est un hydroxyphényle par l'acide acétique glacial en présence d'un excès de dicyclohexylcarbodiimide pour obtenir les dérivés correspondants de formule (I) où $R_3$ est un alcanoyloxy en $C_1$—$C_4$ [et où $Z_1$ est (f)]; ou

(c) la préparation de composés de formule (I), où $R_1$, $R_2$, $NR_1R_2$ et $R_3$ (autre qu'un hydroxyphényle) sont comme précédemment définis, et Z est égal aux fonctions représentées par les lettres (b), (d) et (f) par réaction de:

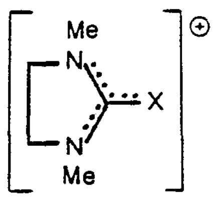

$Y^{\ominus}$

( VI )

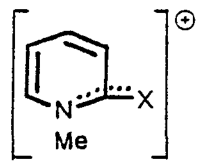

$BF_4^{\ominus}$

( XV )

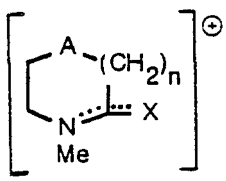

$Y^{\ominus}$

( IXX )

où X est soit un méthoxy, soit un éthoxy, et $Y^{\ominus}$ est soit $BF_4^{\ominus}$, soit $OSO_2F^{\ominus}$ avec un composé de formule:

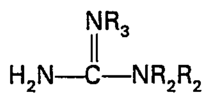

(VII)

pour préparer respectivement un composé de formule:

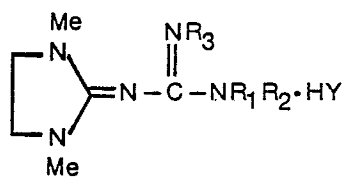

( VIII )

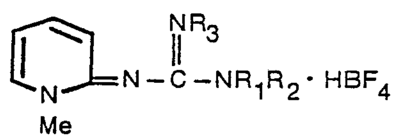

( XVI )

ou

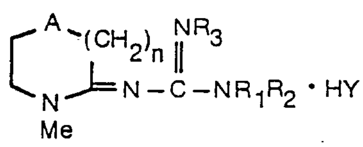

( XX )

et si on le désire, les produits (VIII), (XVI) ou (XX) sous forme du sel correspondant, sont transformés en la base correspondante (I); ou

(d) la préparation de composés de formule (I) où Z est (a), (b) ou (c), Z dans ce cas étant désigné par $Z_2$, caractérisé par la réaction d'un composé de formule:

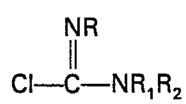

avec un composé de formule $Z_2H$ et, si on le désire, les composés de formule (I) où $R_3$ est un hydroxyphényle peuvent être préparés soit:

(i) par débenzylation des dérivés correspondants où $R_3$ est un benzoyloxyphényle, soit

(ii) par hydrolyse des dérivés correspondants où $R_3$ est un benzyloxyphényle ou un méthoxyphényle.

13. Composition pharmaceutique comprenant un composé selon l'une quelconque des revendications 1 à 11.

14. Composé selon l'une quelconque des revendications 1 à 11, ou composition selon la revendication 13, pour l'emploi comme agent hypoglycémiant.

**Revendications pour l'Etat contractant: AT**

1. Procédé pour préparer un dérivé hétérocyclique de guanidine choisi parmi les composés ayant pour formule:

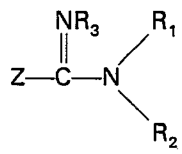

et leurs sels d'addition d'acides acceptables en pharmacie où:

Z est un composant choisi parmi:

(a) 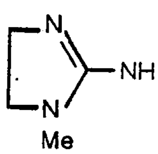 (b) 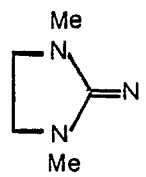 (c) 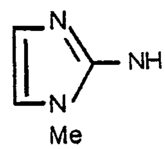

(d) 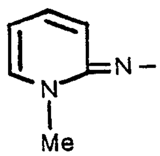 (e) 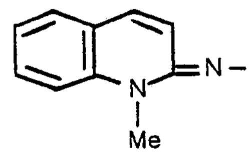 et (f) 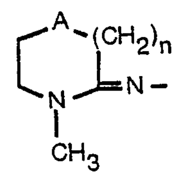

ou:

A est O ou S;

n est zéro ou 1;

$R_1$ est un méthyle ou un éthyle;

$R_2$ est un alkyle en $C_1$—$C_4$, un cycloalkyle ayant 3 à 6 atomes de carbone ou un benzyle;

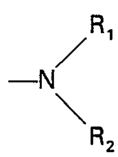

pris ensemble représente:

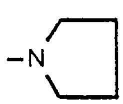 , 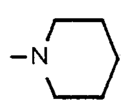 , or 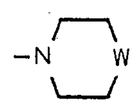

où:

W est O, S, un N-alkyle en $C_1$—$C_4$ ou un N-phényle; et

$R_3$ est un alkyle ayant 4 à 10 atomes de carbone; un phényle; un méthylènedioxyphényle; un phényle substitué par 1 à 3 substituants choisis chacun parmi un halogéno, un alkyle en $C_1$—$C_4$, et un alcoxy en $C_1$—$C_4$; un phényle substitué par un hydroxy, un benzyloxy; un nitro; un trifluorométhyle ou un méthylthio; un naphtyle un cycloalkyle ayant 5 à 8 atomes de carbone; un bicycloalkyle ayant 7 à 10 atomes de carbone; un tricycloalkyle ayant 9 ou 10 atomes de carbone; un arylalkyle dont la fonction aryle est choisie parmi un phényle ou un napthyle et la fonction alkyle a 1 à 4 atomes de carbone; ou un diphénylalkyle dont la fonction alkyle a 1 ou 2 atomes de carbone, caractérisé par:

(a) la réaction d'un composé de formule:

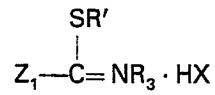

(V)

où $Z_1$ est comme Z mais est outre que (d) ou (e), et dans le cas de (f), A est le soufre et n est zéro; et où $R_3$ est comme précédemment décrit, à l'exception d'un hydrophényle, et dans le cas où $Z_1$ est (f), $R_3$ est comme précédemment décrit, à l'exception d'un hydroxyphényle et d'un alcanoyloxy (en $C_1$—$C_4$) phényle; et

où HX est un sel d'addition d'acide, avec un composé de formule $HNR_1R_2$, où $R_1$, $R_2$ et $NR_1R_2$ sont comme précédemment décrits, pour obtenir les dérivés de guanidine de formule (I) sous forme d'un sel d'addition d'acide, qu'on obtient facilement sous forme de la base correspondante par traitement classique avec un alcali approprié; ou

(b) la préparation de composés de formule (I) où $Z_1$ est (f) et où $R_3$ est un hydroxyphényle, par hydrolyse des dérivés correspondants où $R_3$ est un méthoxyphényle, et acylation des dérivés obtenus où $R_3$ est un hydroxyphényle par l'acide acétique glacial en présence d'un excès de dicyclohexylcarbodiimide pour obtenir les dérivés correspondants de formule (I) où $R_3$ est un alcanoyloxy en $C_1$—$C_4$ [et où $Z_1$ est (f)]; ou

(c) la préparation de composés de formule (I), où $R_1$, $R_2$, $NR_1R_2$ et $R_3$ (autre qu'un hydroxyphényle) sont comme précédemment définis, et Z est égal aux fonctions représentées par les lettres (b), (d) et (f) par réaction de:

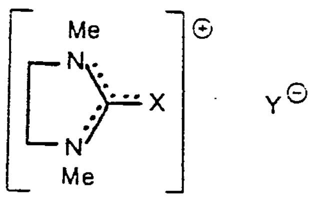

(VI)

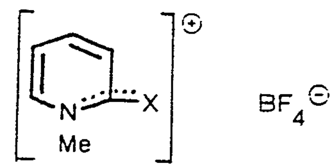

(XV)

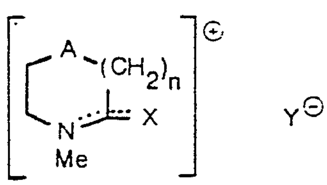

(IXX)

où X est soit un méthoxy, soit un éthoxy, et $Y^{\ominus}$ est soit $BF_4^{\ominus}$, soit $OSO_2F^{\ominus}$ avec un composé de formule:

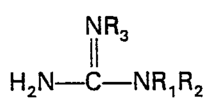

(VII)

pour préparer respectivement un composé de formule:

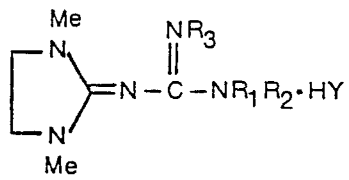

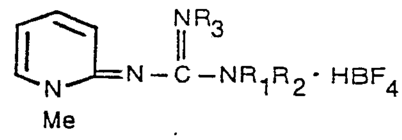

(XVI)

(VIII)

ou

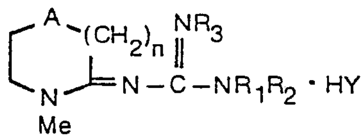

(XX)

et si on le désire, les produits (VIII), (XVI) ou (XX) sous forme du sel correspondant, sont transformés en la base correspondante (I); ou

(d) la préparation de composés de formule (I) où Z est (a), (b) ou (c), Z dans ce cas étant désigné par $Z_2$, caractérisé par la réaction d'un composé de formule:

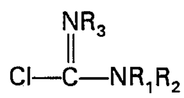

avec un composé de formule $Z_2H$ et, si on le désire, les composés de formule (I) où $R_3$ est un hydroxyphényle peuvent être préparés soit:

(i) par débenzylation des dérivés correspondants où $R_3$ est un benzoyloxyphényle, soit

(ii) par hydrolyse des dérivés correspondants où $R_3$ est un benzyloxyphényle ou un méthoxyphényle.

2. Procédé selon la revendication 2, pour la préparation d'un composé choisi parmi le N-(1-méthyl-2-imidazolidinylidène)-N'-phényl-1-pyrrolidinecarboximidamide et sels sels d'addition d'acides acceptables en pharmacie.

3. Procédé selon la revendication 1, pour la préparation d'un composé choisi parmi le N-(1,3-diméthyl-2-imidazolidinylidène)-N'-phényl-1-pyrrolidinecarboximidamide et ses sels d'addition d'acides acceptables en pharmacie.

4. Procédé selon la revendication 1, pour la préparation d'un composé choisi parmi le N-(2-méthylimidazol-2-yl)-N'-phényl-4-morpholinecarboximidamide et ses sels d'addition d'acides acceptables en pharmacie.

5. Procédé selon la revendication 1, pour la préparation d'un composé choisi parmi le N-(1-amadantyl)-N'-(1-méthylimidazol-2-yl)-1-pyrrolidinecarboximidamide et ses sels d'addition d'acides acceptables en pharmacie.

6. Procédé selon la revendication 1, pour la préparation d'un composé choisi parmi le N-(1,2-dihydro-1-méthyl-2(1*H*)pyridinylidène)-N'-phényl-1-pyrrolidinecarboximidamide et ses sels d'addition d'acides acceptables en pharmacie.

7. Procédé selon la revendication 1, pour la préparation d'un composé choisi parmi le N-(1,2-dihydro-1-méthyl-2(1H)quinoléinylidène)-N'-phényl-1-pyrrolidinecarboximidamide et ses sels d'addition d'acides acceptables en pharmacie.

8. Procédé selon la revendication 1, pour la préparation d'un composé choisi parmi le N-(3-méthyl-2-thiazolidinylidène)-N'-phényl-1-pyrrolidinecarboximidamide et ses sels d'addition d'acides acceptables en pharmacie.

9. Procédé selon la revendication 1, pour la préparation d'un composé choisi parmi le N-(3-méthyl-2-oxazolidinylidène)-N'-phényl-1-pyrrolidinecarboximidamide et ses sels d'addition d'acides acceptables en pharmacie.

10. Procédé selon la revendication 1, pour la préparation d'un composé choisi parmi le N-(2,3,5,6-tétrahydro-4-méthyl-1,4-oxazine-3-ylidène)-N'-phényl-1-pyrrolidinecarboximidamide et ses sels d'addition d'acides acceptables en pharmacie.

11. Procédé selon la revendication 1, pour la préparation d'un composé choisi parmi le N[(2,3,5,6-tétrahydro-4-méthyl)-1,4-thiazin-3-ylidène]-N'-(4'-méthoxyphényl)-1-pipéridinecarboximidamide et ses sels d'addition d'acides acceptables en pharmacie.

12. Procédé pour la préparation d'une composition pharmaceutique, caractérisé par l'incorporation d'un composé obtenu selon un procédé selon l'une quelconque des revendications 1 à 11 à des formes d'administration et préparations pharmaceutiques liquides et solides classiques.